# EUROPEAN PATENT APPLICATION

(11) **EP 4 610 877 A1**
(43) Date of publication of application: **03.09.2025**
(21) Application number: 25160467.4
(22) Date of filing: 27.02.2025
(51) Int. Cl.: G06F 40/216, G06F 40/30

(54) **SYSTEM AND COMPUTER-IMPLEMENTED METHOD FOR EVALUATING GENERATED CLINICAL NOTES AND NOTE GENERATION MODELS**

(30) Priority: 01.03.2024 US 202463560083 P
(71) Applicant: Solventum Intellectual Properties Company, Maplewood, MN 55144 (US)
(72) Inventor: Glover, John, Dublin, A94 FD30 (IE); Schaaf, Thomas, Pittsburgh, 15208 (US); Kadkhodaie Elyaderani, Mojtaba, Saint Paul, 55116 (US); Lucke, Michael S., White Bear Township, 55127 (US)
(74) Representative: Vossius & Partner Patentanwälte Rechtsanwälte mbB

(57) **Abstract**

A first system includes a processor configured to: compare, via a machine learning model, a generated clinical note and a plurality of pseudo-reference clinical notes based on at least one evaluation metric to predict a quality score of the generated clinical note; and output, via an output device, the generated clinical note if the quality score is greater than or equal to a predetermined acceptable threshold, and a request to manually draft a clinical note otherwise. A second system includes a processor configured to: compare, via a machine learning model, a plurality of generated clinical notes and at least one final clinical note based on a plurality of evaluation metrics to determine model scores associated with the note generation models that generated the plurality of generated clinical notes; and select one of the plurality of note generation models at least partially based on the model scores.

## Description

### Technical Field

The present disclosure generally relates to a system and a computer-implemented method for evaluating generated clinical notes, and a system and a computer-implemented method for evaluating a plurality of note generation models that generate clinical notes.

### Background

Generated clinical notes, which are produced by note generation models, may have varying quality depending upon inputs to the note generation models and training of the note generation models. A low quality generated clinical note may interrupt workflow, as a large number of edits may be required to bring the low quality generated clinical note to an acceptable quality. Therefore, there is a need for systems and computer-implemented methods for evaluating generated clinical notes to predict their quality and for evaluating note generation models that generate clinical notes.

### Summary

In a first aspect, the present disclosure provides a system for evaluating generated clinical notes. The system includes one or more computer processors. The system further includes at least one non-transitory computer-readable storage communicatively coupled to the one or more computer processors. The at least one non-transitory computer-readable storage has stored thereon a note generation model, a machine learning model, and instructions that, when executed by the one or more computer processors, cause the one or more computer processors to receive a generated clinical note associated with a patient and a doctor-patient conversation (DoPaCo) transcript based on which the generated clinical note is generated. The instructions further cause the one or more computer processors to provide the DoPaCo transcript to the note generation model. The instructions further cause the one or more computer processors to generate, via the note generation model, a plurality of pseudo-reference clinical notes based on the DoPaCo transcript. The instructions further cause the one or more computer processors to select at least one evaluation metric for evaluating the generated clinical note. The instructions further cause the one or more computer processors to extract, via the machine learning model, for each evaluation metric from the at least one evaluation metric, a plurality of generated units from the generated clinical note. The instructions further cause the one or more computer processors to extract, via the machine learning model, for each evaluation metric, a plurality of sets of pseudo units from the plurality of pseudo-reference clinical notes. Each set of pseudo units from the plurality of sets of pseudo units is associated with a respective pseudo-reference clinical note from the plurality of pseudo-reference clinical notes. Each set of pseudo units includes a plurality of pseudo units. The instructions further cause the one or more computer processors to compare, via the machine learning model, for each evaluation metric, the plurality of generated units and the plurality of pseudo units of each set of pseudo units corresponding to the evaluation metric. The instructions further cause the one or more computer processors to calculate, for each evaluation metric, a plurality of sub-scores for the generated clinical note based on the comparison of the plurality of generated units and the plurality of pseudo units of each set of pseudo units corresponding to the evaluation metric. Each sub-score from the plurality of sub-scores corresponding to a respective evaluation metric is associated with a respective pseudo-reference clinical note. The instructions further cause the one or more computer processors to aggregate, for each evaluation metric, the plurality of sub-scores corresponding to the evaluation metric to calculate a metric score corresponding to the evaluation metric. The instructions further cause the one or more computer processors to aggregate, via the machine learning model, the metric score corresponding to each of the at least one evaluation metric to predict a quality score of the generated clinical note. The instructions further cause the one or more computer processors to output, via an output device, the generated clinical note if the quality score is greater than or equal to a predetermined acceptable threshold. The instructions further cause the one or more computer processors to output, via the output device, a request to manually draft a drafted clinical note based on the DoPaCo transcript if the quality score is less than the predetermined acceptable threshold.

In a second aspect, the present disclosure provides a computer-implemented method for evaluating generated clinical notes. The computer-implemented method includes receiving a generated clinical note associated with a patient and a doctor-patient conversation (DoPaCo) transcript based on which the generated clinical note is generated. The computer-implemented method further includes providing the DoPaCo transcript to a note generation model. The computer-implemented method further includes generating, via the note generation model, a plurality of pseudo-reference clinical notes based on the DoPaCo transcript. The computer-implemented method further includes selecting at least one evaluation metric for evaluating the generated clinical note. The computer-implemented method further includes extracting, via a machine learning model, for each evaluation metric from the at least one evaluation metric, a plurality of generated units from the generated clinical note. The computer-implemented method further includes extracting, via the machine learning model, for each evaluation metric, a plurality of sets of pseudo units from the plurality of pseudo-reference clinical notes. Each set of pseudo units from the plurality of sets of pseudo units is associated with a respective pseudo-reference clinical note from the plurality of pseudo-reference clinical notes. Each set of pseudo units includes a plurality of pseudo units. The computer-implemented method further includes comparing, via the machine learning model, for each evaluation metric, the plurality of generated units and the plurality of pseudo units of each set of pseudo units corresponding to the evaluation metric. The computer-implemented method further includes calculating, for each evaluation metric, a plurality of sub-scores for the generated clinical note based on the comparison of the plurality of generated units and the plurality of pseudo units of each set of pseudo units corresponding to the evaluation metric. Each sub-score from the plurality of sub-scores corresponding to a respective evaluation metric is associated with a respective pseudo-reference clinical note. The computer-implemented method further includes aggregating, for each evaluation metric, the plurality of sub-scores corresponding to the evaluation metric to calculate a metric score corresponding to the evaluation metric. The computer-implemented method further includes aggregating, via the machine learning model, the metric score corresponding to each of the at least one evaluation metric to predict a quality score of the generated clinical note. The computer-implemented method further includes outputting, via an output device, the generated clinical note if the quality score is greater than or equal to a predetermined acceptable threshold. The computer-implemented method further includes outputting, via the output device, a request to manually draft a drafted clinical note based on the DoPaCo transcript if the quality score is less than the predetermined acceptable threshold.

In a third aspect, the present disclosure provides a system for evaluating note generation models that generate clinical notes. The system includes one or more computer processors. The system further includes at least one non-transitory computer-readable storage communicatively coupled to the one or more computer processors. The at least one non-transitory computer-readable storage has stored thereon a machine learning model and instructions that when executed by the one or more computer processors cause the one or more computer processors to receive a plurality of generated clinical notes. The plurality of generated clinical notes is generated by a corresponding plurality of note generation models based on a doctor-patient conversation (DoPaCo) transcript. The plurality of note generation models includes a production note generation model that is currently used to generate clinical notes. The plurality of generated clinical notes includes a production clinical note that is generated by the production note generation model. The instructions further cause the one or more processors to receive at least one final clinical note that is approved by a medical professional based on the DoPaCo transcript. The instructions further cause the one or more processors to provide the plurality of generated clinical notes and the at least one final clinical note to the machine learning model. The instructions further cause the one or more processors to select a plurality of evaluation metrics for evaluating the plurality of generated clinical notes. The instructions further cause the one or more processors to extract, via the machine learning model, for each evaluation metric from the plurality of evaluation metrics, a plurality of sets of generated units from the plurality of generated clinical notes. Each set of generated units from the plurality of sets of generated units is associated with a respective generated clinical note from the plurality of generated clinical notes. Each set of generated units includes a plurality of generated units. The instructions further cause the one or more processors to extract, via the machine learning model, for each evaluation metric, a plurality of final units from the at least one final clinical note. The instructions further cause the one or more processors to compare, via the machine learning model, for each evaluation metric, the plurality of generated units of each set of generated units and the plurality of final units of the at least one final clinical note corresponding to the evaluation metric. The instructions further cause the one or more processors to calculate, for each evaluation metric, a metric score for each of the plurality of generated clinical notes based on the comparison of the plurality of generated units of each set of generated units and the plurality of final units of the at least one final clinical note corresponding to the evaluation metric. The instructions further cause the one or more processors to aggregate, via the machine learning model, for each generated clinical note, the metric scores corresponding to the plurality of evaluation metrics to determine a model score associated with the note generation model that generated the generated clinical note. The instructions further cause the one or more processors to select one of the plurality of note generation models to generate clinical notes at least partially based on the model scores.

In a fourth aspect, the present disclosure provides a computer-implemented method for evaluating note generation models that generate clinical notes. The computer-implemented method includes receiving a plurality of generated clinical notes. The plurality of generated clinical notes is generated by a corresponding plurality of note generation models based on a doctor-patient conversation (DoPaCo) transcript. The plurality of note generation models includes a production note generation model that is currently used to generate clinical notes. The plurality of generated clinical notes includes a production clinical note that is generated by the production note generation model. The computer-implemented method further includes receiving at least one final clinical note that is approved by a medical professional based on the DoPaCo transcript. The computer-implemented method further includes providing the plurality of generated clinical notes and the at least one final clinical note to a machine learning model. The computer-implemented method further includes selecting a plurality of evaluation metrics for evaluating the plurality of generated clinical notes. The computer-implemented method further includes extracting, via the machine learning model, for each evaluation metric from the plurality of evaluation metrics, a plurality of sets of generated units from the plurality of generated clinical notes. Each set of generated units from the plurality of sets of generated units is associated with a respective generated clinical note from the plurality of generated clinical notes. Each set of generated units includes a plurality of generated units. The computer-implemented method further includes extracting, via the machine learning model, for each evaluation metric, a plurality of final units from the at least one final clinical note. The computer-implemented method further includes comparing, via the machine learning model, for each evaluation metric, the plurality of generated units of each set of generated units and the plurality of final units of the at least one final clinical note corresponding to the evaluation metric. The computer-implemented method further includes calculating, for each evaluation metric, a metric score for each of the plurality of generated clinical notes based on the comparison of the plurality of generated units of each set of generated units and the plurality of final units of the at least one final clinical note corresponding to the evaluation metric. The computer-implemented method further includes aggregating, via the machine learning model, for each generated clinical note, the metric scores corresponding to the plurality of evaluation metrics to determine a model score associated with the note generation model that generated the generated clinical note. The computer-implemented method further includes selecting one of the plurality of note generation models to generate clinical notes at least partially based on the model scores.

The details of one or more examples of the disclosure are set forth in the accompanying drawings and the description below. Other features, objects, and advantages of the disclosure will be apparent from the description and drawings, and from the claims.

### Brief Description of the Drawings

Exemplary embodiments disclosed herein may be more completely understood in consideration of the following detailed description in connection with the following figures. The figures are not necessarily drawn to scale. Like numbers used in the figures refer to like components. However, it will be understood that the use of a number to refer to a component in a given figure is not intended to limit the component in another figure labeled with the same number.
FIG. 1 is a schematic block diagram of a system according to an embodiment of the present disclosure;
FIG. 2 is a schematic block diagram of a system for evaluating generated clinical notes according to an embodiment of the present disclosure;
FIG. 3A is a schematic block diagram depicting a generated clinical note and a plurality of generated units extracted from the generated clinical note according to an embodiment of the present disclosure;
FIG. 3B is a schematic block diagram depicting a plurality of pseudo-reference clinical notes and a plurality of sets of pseudo units extracted from the plurality of pseudo-reference clinical notes according to an embodiment of the present disclosure;
FIG. 4 is a schematic block diagram depicting a plurality of sets of sub-scores calculated for a plurality of evaluation metrics, a plurality of metric scores for the plurality of evaluation metrics, and a quality score of the generated clinical note according to an embodiment of the present disclosure;
FIG. 5A is a schematic block diagram depicting a Doctor-Patient Conversation (DoPaCo) transcript, a plurality of transcript confidence scores corresponding to a plurality of transcript units of the DoPaCo transcript, and a plurality of transcript weights assigned to the plurality of generated units according to an embodiment of the present disclosure;
FIG. 5B is a schematic block diagram depicting a knowledge graph and a plurality of knowledge weights assigned to the plurality of generated units according to an embodiment of the present disclosure;
FIG. 6 is a schematic block diagram depicting the generated clinical note, the plurality of generated units extracted from the generated clinical note, and a plurality of unit scores of the plurality of generated units according to an embodiment of the present disclosure;
FIG. 7A is a schematic block diagram of the generated clinical note depicting a plurality of generated note sections thereof according to an embodiment of the present disclosure;
FIG. 7B is a schematic block diagram of a pseudo reference-clinical note depicting a plurality of pseudo note sections thereof according to an embodiment of the present disclosure;
FIG. 8 is a schematic block diagram depicting a plurality of pseudo-reference clinical notes, a plurality of section sub-scores of the plurality of generated note sections, and a plurality of section scores according to an embodiment of the present disclosure;
FIG. 9 is a schematic block diagram depicting a drafted clinical note, a plurality of drafted units extracted from the drafted clinical note, and a draft reference metric score according to an embodiment of the present disclosure;
FIG. 10 is a schematic block diagram depicting a plurality of draft reference metric scores for a plurality of evaluation metrics, and a draft reference quality score of the drafted clinical note according to an embodiment of the present disclosure;
FIG. 11 is a schematic block diagram depicting an amended clinical note, a plurality of amended units extracted from the amended clinical note, and an amended reference metric score according to an embodiment of the present disclosure;
FIG. 12 is a schematic block diagram depicting a plurality of amended reference metric scores for a plurality of evaluation metrics, and an amended reference quality score of the amended clinical note according to an embodiment of the present disclosure;
FIG. 13 is flowchart depicting various steps of a computer-implemented method for evaluating generated clinical notes according to an embodiment of the present disclosure;
FIG. 14 is a schematic block diagram of a system for evaluating note generation models that generate clinical notes according to an embodiment of the present disclosure;
FIG. 15A is a schematic block diagram depicting a plurality of generated clinical notes and a plurality of sets of generated units extracted from the plurality of generated clinical notes according to an embodiment of the present disclosure;
FIG. 15B is a schematic block diagram depicting a final clinical note and a plurality of final units extracted from the final clinical note according to an embodiment of the present disclosure;
FIG. 16 is a schematic block diagram depicting a plurality of metric scores corresponding to a plurality of evaluation metrics calculated for each generated clinical note, and model scores for note generation models that generated the generated clinical notes according to an embodiment of the present disclosure;
FIG. 17A is a schematic block diagram depicting a plurality of second generated clinical notes and a plurality of sets of second generated units extracted from the plurality of second generated clinical notes according to an embodiment of the present disclosure;
FIG. 17B is a schematic block diagram depicting a second final clinical note and a plurality of second final units extracted from the second final clinical note according to an embodiment of the present disclosure;
FIG. 18 is a schematic block diagram depicting a plurality of second metric scores corresponding to a plurality of evaluation metrics calculated for each second generated clinical note, second model scores for note generation models that generated the second generated clinical notes, and aggregated model scores for the note generation models according to an embodiment of the present disclosure;
FIG. 19 is flowchart depicting various steps of a computer-implemented method for evaluating note generation models that generate clinical notes according to an embodiment of the present disclosure;
FIG. 20A is a flow diagram depicting steps for determining a metric score according to an embodiment of the present disclosure; and
FIG. 20B is a flow diagram depicting steps for determining a metric score according to another embodiment of the present disclosure.

### Detailed Description

In the following description, reference is made to the accompanying figures that form a part thereof and in which various embodiments are shown by way of illustration. It is to be understood that other embodiments are contemplated and may be made without departing from the scope or spirit of the present disclosure. The following detailed description, therefore, is not to be taken in a limiting sense.

In the following disclosure, the following definitions are adopted.

As recited herein, all numbers should be considered modified by the term "about." As used herein, "a," "an," "the," "at least one," and "one or more" are used interchangeably.

As used herein as a modifier to a property or attribute, the term "generally," unless otherwise specifically defined, means that the property or attribute would be readily recognizable by a person of ordinary skill but without requiring absolute precision or a perfect match (e.g., within +/- 20 % for quantifiable properties).

The term "substantially," unless otherwise specifically defined, means to a high degree of approximation (e.g., within +/- 10% for quantifiable properties) but again without requiring absolute precision or a perfect match.

The term "about," unless otherwise specifically defined, means to a high degree of approximation (e.g., within +/- 5% for quantifiable properties) but again without requiring absolute precision or a perfect match.

Terms such as same, equal, uniform, constant, strictly, and the like, are understood to be within the usual tolerances or measuring error applicable to the particular circumstance rather than requiring absolute precision or a perfect match.

As used herein, when a first material is termed as "similar" to a second material, at least 90% by weight of the first and second materials are identical and any variation between the first and second materials comprises less than about 10% by weight of each of the first and second materials.

As used herein, "at least one of A and B" and "at least one of A or B" should be understood to mean "only A, only B, or both A and B."

As used herein, the term "configured to" and like is at least as restrictive as the term "adapted to" and requires actual design intention to perform the specified function rather than mere physical capability of performing such a function.

As used herein, the terms "medical" and "clinical" are interchangeable and have the same meaning.

As used herein, the term "patient," and its equivalents, refers to an individual being monitored and/or cared for within a clinical environment or who has been previously monitored and/or cared for within the clinical environment. In various examples, a patient is a human, but implementations of this disclosure are not limited thereto. Examples of the clinical environment may include, but are not limited to, a doctor's office, a medical facility, a medical practice, a medical lab, an urgent care facility, a medical clinic, an emergency room, an operating room, a hospital, a long term care facility, a rehabilitation facility, a nursing home, and a hospice facility.

As used herein, the term "medical professional" refers to any person working in the healthcare industry, such as doctors, nurses, physician's assistants, lab technicians, physical therapists, scribes (e.g., a transcriptionist), and the like.

As used herein, the term "electronic health record" or "EHR" refers to a database that stores clinical notes and other clinical data related to a patient. EHR may be queried to receive the clinical notes and the other clinical data of the patient.

As used herein, the term "clinical note" refers to a note including clinical data of a patient that is generated based on an interaction between the patient and a medical professional. Clinical notes may be stored in an electronic format (e.g., a text document), typically in an electronic health record (EHR).

As used herein, the term "transcript" refers to data representing a conversation in any suitable data format. For example, a transcript may be in the form of text, audio, video, etc. The term "doctor-patient conversation transcript" or "DoPaCo transcript" refers to data representing a conversation between a doctor and a patient.

As used herein, the term "clinical concept" represents any attribute of a patient. Such attributes include, but are not limited to, a chief complaint of the patient, a history of present illness of the patient, a past medical history of the patient, a social history of the patient, a family history of the patient, a review of systems of the patient, allergies of the patient, medications of the patient, impressions of the patient by a clinician, a medical plan for the patient, diagnostic imaging results preformed the patient, results of a medical test of the patient, a gender of the patient, an ethnicity of the patient, an age of the patient, a physical attribute of the patient, physical signs of the patient, physical systems of the patient, a time period associated with one of the preceding attributes or another attribute, and/or other attributes.

As used herein, the term "score" refers to a value calculated or predicted to represent a degree of similarity between two sets of data. Scores may be characterized using various conventions. One example includes a numerical value ranging from 0 to 1.

As used herein, the term "processor" or "computer processor" refers any device that performs logic operations. A computer processor may include a general processor, a central processing unit, an application specific integrated circuit (ASIC), a digital signal processor, a field programmable gate array (FPGA), a digital circuit, an analog circuit, a controller, a microcontroller, any other type of processor, or any combination thereof.

As used herein, the term "instructions" refers to code (e.g., source code, compiled code, code that can be interpreted, executable code, etc.) that, when executed by a processor, causes the processor to perform various steps, functions, operations, and/or calculations, i.e., the conventional meaning of the term "instructions" with respect to digital technology.

As used herein, the term "storage device" refers to any storage medium that is capable of storing data and information in an electronic format. Examples of a storage device include hard drives, flash drives, optical media, and the like.

As used herein, the term "communicatively coupled" refers to any type of connection or coupling that allows for the exchange or sharing of information. Two communicatively coupled components may be electrically coupled by, for example, a wire; optically coupled by, for example, an optical cable; and/or wirelessly coupled by, for example, a radio frequency or other transmission media. Two communicatively coupled components may be directly coupled, or indirectly coupled, such as via a network.

As used herein, the term "model," "machine learning model," or "ML model" refers to a machine learning algorithm or collection of algorithms that takes structured and/or unstructured data inputs and generates a prediction or result. That is, a machine learning model may be a computer model or a computer representation that may be tuned (e.g., trained) based on inputs to approximate unknown functions. The process of building or optimizing a machine learning model is referred to herein as "training." Examples of machine-learning models include, for example, one or more of vectorization machine-learning models, sequence-to-sequence models, transformer models, a decision tree (e.g., a gradient boosted decision tree), a linear regression model, a logistic regression model, association rule learning, inductive logic programming, support vector learning, a Bayesian network, a regression-based model, a neural network, or combinations thereof.

As used herein, the term "neural network" may refer to one example of a machine learning model that can be tuned (e.g., trained) based on inputs to approximate unknown functions. In particular, the neural network may include a model of interconnected neurons (arranged in layers) that communicate and learn to approximate complex functions and generate outputs based on a plurality of inputs provided to the model. For example, the neural network may include deep neural network (DNN), deep convolutional neural networks (CNN), Region-CNN (R-CNN), Faster R-CNN, Mask R-CNN, fully convolutional neural networks, recurrent neural networks ("RNNs"), such as long short-term memory neural networks ("LSTMs"), graph neural networks, generative adversarial neural networks (GAN), and single-shot detect (SSD) networks. In other words, a neural network is an algorithm that implements deep learning techniques, which utilize a set of learned parameters arranged in layers according to a particular architecture to attempt to model high-level abstractions in data using supervisory data to tune parameters of the neural network.

As used herein, the term "knowledge graph" refers to a networked data structure including facts that are represented in nodes and edges that represent connections or links between the nodes. A knowledge graph may represent a knowledge base of data, i.e., facts, and their semantic relationships. A knowledge graph for a certain topic may be generated from a knowledge corpus covering the topic. For example, a knowledge graph of clinical data associated with a patient may be generated from historical clinical or medical records of the patient.

As used herein, the term "quality" in conjunction with a clinical note refers to an accuracy of the clinical note with respect to a doctor-patient conversation. The quality of a clinical note may represent a time and/or an effort it will take to edit or amend the clinical note to bring it to an acceptable quality. A high quality note may require less or no edits, and therefore less time and effort to bring it to the acceptable quality, while a low quality note may require a large number of edits, and therefore more time and effort to bring it to the acceptable quality.

In a first aspect, the present disclosure relates to a system for evaluating generated clinical notes. The system includes one or more computer processors. The system further includes at least one non-transitory computer-readable storage communicatively coupled to the one or more computer processors. The at least one non-transitory computer-readable storage has stored thereon a note generation model, a machine learning model, and instructions that, when executed by the one or more computer processors, cause the one or more computer processors to receive a generated clinical note associated with a patient and a doctor-patient conversation (DoPaCo) transcript based on which the generated clinical note is generated. The instructions further cause the one or more computer processors to provide the DoPaCo transcript to the note generation model. The instructions further cause the one or more computer processors to generate, via the note generation model, a plurality of pseudo-reference clinical notes based on the DoPaCo transcript. The instructions further cause the one or more computer processors to select at least one evaluation metric for evaluating the generated clinical note. The instructions further cause the one or more computer processors to extract, via the machine learning model, for each evaluation metric from the at least one evaluation metric, a plurality of generated units from the generated clinical note. The instructions further cause the one or more computer processors to extract, via the machine learning model, for each evaluation metric, a plurality of sets of pseudo units from the plurality of pseudo-reference clinical notes. Each set of pseudo units from the plurality of sets of pseudo units is associated with a respective pseudo-reference clinical note from the plurality of pseudo-reference clinical notes. Each set of pseudo units includes a plurality of pseudo units. The instructions further cause the one or more computer processors to compare, via the machine learning model, for each evaluation metric, the plurality of generated units and the plurality of pseudo units of each set of pseudo units corresponding to the evaluation metric. The instructions further cause the one or more computer processors to calculate, for each evaluation metric, a plurality of sub-scores for the generated clinical note based on the comparison of the plurality of generated units and the plurality of pseudo units of each set of pseudo units corresponding to the evaluation metric. Each sub-score from the plurality of sub-scores corresponding to a respective evaluation metric is associated with a respective pseudo-reference clinical note. The instructions further cause the one or more computer processors to aggregate, for each evaluation metric, the plurality of sub-scores corresponding to the evaluation metric to calculate a metric score corresponding to the evaluation metric. The instructions further cause the one or more computer processors to aggregate, via the machine learning model, the metric score corresponding to each of the at least one evaluation metric to predict a quality score of the generated clinical note. The instructions further cause the one or more computer processors to output, via an output device, the generated clinical note if the quality score is greater than or equal to a predetermined acceptable threshold. The instructions further cause the one or more computer processors to output, via the output device, a request to manually draft a drafted clinical note based on the DoPaCo transcript if the quality score is less than the predetermined acceptable threshold.

The system of the first aspect may employ the machine learning model to predict the quality score of the generated clinical note, which may correlate with an effort required to improve/correct the generated clinical note to an acceptable quality level. Specifically, the note generation model generates the plurality of pseudo-reference clinical notes, which is utilized by the machine learning model as missing human-written notes (since there are no human-written notes available in a production environment) corresponding to the DoPaCo transcript.

Further, the system of the first aspect may evaluate the generated clinical note based on a plurality of evaluation metrics. Each evaluation metric may be related to an aspect of errors that contribute to extra effort/time required for correcting the generated clinical note. Therefore, evaluating the generated clinical note based on the plurality of evaluation metrics may encompass various aspects that contribute to extra effort/time for correcting the generated clinical note and reduce metric bias of each individual evaluation metric.

In some examples, the system of the first aspect may also provide explanations for the predicted quality score and hints for how the generated clinical note can be improved, for example, by highlighting portions of the generated clinical note that are predicted to be of low quality and adding missing data to the generated clinical note.

In some examples, the system of the first aspect may employ quality prediction in a section-by-section manner. That is, the plurality of generated units of one section of the generated clinical note may be compared with the plurality of pseudo units of each of the plurality of pseudo-reference clinical notes of the same section, rather than comparing the generated clinical note and the plurality of pseudo-reference clinical notes in their entirety. The sections having a low section score may be removed from the generated clinical note. Alternatively, and according to particular implementations, sections having a high score (based, e.g., on a separate and/or independent note generation model) can be selected for inclusion in the generated clinical note.

In a second aspect, the present disclosure further relates to a system for evaluating note generation models that generate clinical notes. The system includes one or more computer processors. The system further includes at least one non-transitory computer-readable storage communicatively coupled to the one or more computer processors. The at least one non-transitory computer-readable storage has stored thereon a machine learning model and instructions that when executed by the one or more computer processors cause the one or more computer processors to receive a plurality of generated clinical notes. The plurality of generated clinical notes is generated by a corresponding plurality of note generation models based on a doctor-patient conversation (DoPaCo) transcript. The plurality of note generation models includes a production note generation model that is currently used to generate clinical notes. The plurality of generated clinical notes includes a production clinical note that is generated by the production note generation model. The instructions further cause the one or more processors to receive at least one final clinical note that is approved by a medical professional based on the DoPaCo transcript. The instructions further cause the one or more processors to provide the plurality of generated clinical notes and the at least one final clinical note to the machine learning model. The instructions further cause the one or more processors to select a plurality of evaluation metrics for evaluating the plurality of generated clinical notes. The instructions further cause the one or more processors to extract, via the machine learning model, for each evaluation metric from the plurality of evaluation metrics, a plurality of sets of generated units from the plurality of generated clinical notes. Each set of generated units from the plurality of sets of generated units is associated with a respective generated clinical note from the plurality of generated clinical notes. Each set of generated units includes a plurality of generated units. The instructions further cause the one or more processors to extract, via the machine learning model, for each evaluation metric, a plurality of final units from the at least one final clinical note. The instructions further cause the one or more processors to compare, via the machine learning model, for each evaluation metric, the plurality of generated units of each set of generated units and the plurality of final units of the at least one final clinical note corresponding to the evaluation metric. The instructions further cause the one or more processors to calculate, for each evaluation metric, a metric score for each of the plurality of generated clinical notes based on the comparison of the plurality of generated units of each set of generated units and the plurality of final units of the at least one final clinical note corresponding to the evaluation metric. The instructions further cause the one or more processors to aggregate, via the machine learning model, for each generated clinical note, the metric scores corresponding to the plurality of evaluation metrics to determine a model score associated with the note generation model that generated the generated clinical note. The instructions further cause the one or more processors to select one of the plurality of note generation models to generate clinical notes at least partially based on the model scores.

The system of the second aspect may evaluate the plurality of note generation models based on the plurality of evaluation metrics with respect to the at least one final clinical note. In some examples, the at least one final clinical note may include a plurality of final clinical notes approved by a corresponding plurality of medical professionals. This may allow aggregating the model scores determined for the plurality of final clinical notes. Using the plurality of final clinical notes for evaluation may allow a more comprehensive evaluation of the plurality of note generation models, as different medical professionals may produce clinical notes in various different manners (writing style, formatting, terminology used, etc.).

In examples where the at least one final clinical note is produced by amending the production clinical note, some metrics may exhibit a bias towards the production note generation model, that is, the model score for the production note generation model may be high. Content factuality metric and the content coverage metric, as disclosed in the present disclosure, may be less biased than others of the plurality of evaluation metrics for evaluation in such cases, and therefore may be preferably assigned higher aggregation weights for determining the model score for each note generation model. Thus, in such cases, it may be beneficial to rely more heavily on evaluation metrics such as the content factuality metric and the content coverage metric to obtain accurate model scores. In some examples, the system of the second aspect may select the note generation model that is best suited for a medical professional based on their preference.

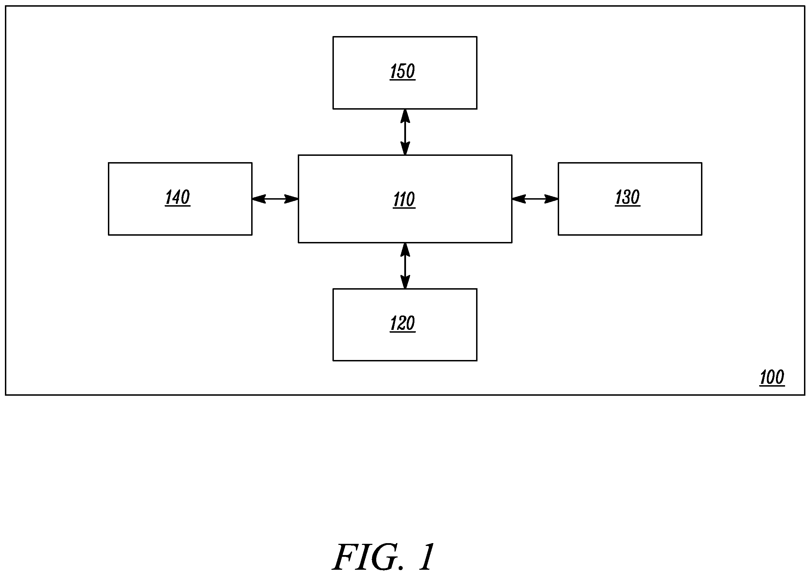

Referring now to the Figures, FIG. 1 illustrates a schematic block diagram of a system 100 according to an embodiment of the present disclosure.

The system 100 includes one or more computer processors 110 (hereinafter referred to as "the processor 110"). The processor 110 may include, for example, a general processor, a central processing unit, an application specific integrated circuit (ASIC), a digital signal processor, a field programmable gate array (FPGA), a digital circuit, an analog circuit, a controller, a microcontroller, any other type of processor, or any combination thereof.

The system 100 further includes at least one non-transitory computer-readable storage 120 (hereinafter referred to as "the non-transitory storage 120") communicatively coupled to the processor 110. The non-transitory storage 120 may include any type of physical memory on which information or data readable by the processor 110 can be stored. Examples of the non-transitory storage 120 include Random Access Memory (RAM), Read-Only Memory (ROM), volatile memory, nonvolatile memory, hard drives, CD ROMs, DVDs, flash drives, disks, any other optical data storage medium, a PROM, an EPROM, a FLASH-EPROM or any other flash memory, NVRAM, a cache, a register, any other memory chip or cartridge, and networked versions of the same.

The processor 110 may be further communicatively coupled to a storage device 130. The storage device 130 may include any type of physical memory on which information or data readable by the processor 110 can be stored. In some examples, the system 100 may include the storage device 130. In some examples, the storage device 130 may be disposed remote from the processor 110 and communicatively coupled to the processor 110 via a network.

The processor 110 may be further communicatively coupled to an output device 140. The processor 110 may display one or more outputs via the output device 140, for example, in the form of a user interface. The output device 140 may include, but is not limited to, a monitor.

The processor 110 may be further communicatively coupled to an input device 150. The input device 150 may enable a user, such as a medical professional, to provide inputs to the processor 110. Specifically, the input device 150 may enable the user to interact with the user interface displayed via the output device 140. The input device 150 may include, but is not limited to, a mouse, a keyboard, and so forth.

The system 100 may be configured to perform computer-implemented methods of the present disclosure. Specifically, the processor 110 of the system 100 may execute instructions that cause the processor 110 to perform various steps of the computer-implemented methods of the present disclosure.

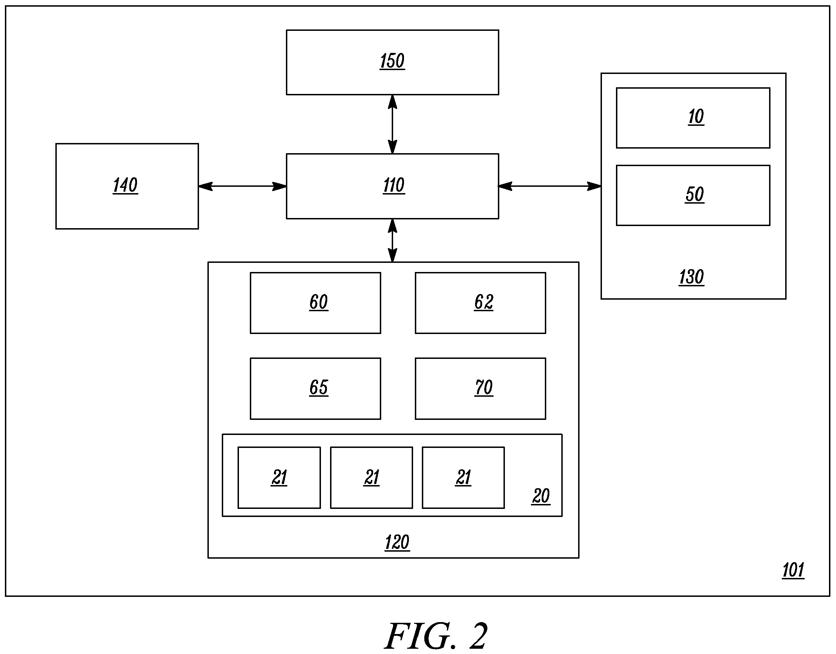

FIG. 2 illustrates a system 101 for evaluating generated clinical notes according to an embodiment of the present disclosure. The system 101 is similar to the system 100 of FIG. 1, with like elements designated by like reference characters.

Referring to FIG. 2, the non-transitory storage 120 has stored thereon a note generation model 60, a machine learning model 62, and instructions 65. In other words, the note generation model 60, the machine learning model 62 and the instructions 65 are stored on the non-transitory storage 120.

In some examples, the storage device 130 may store a generated clinical note 10 associated with a patient. The generated clinical note 10 is "AI generated," i.e., generated or produced by a note generation model based on a doctor-patient conversation (DoPaCo) transcript 50. The note generation model that produced the generated clinical note 10 is hereinafter referred to as "the production note generation model." The production note generation model may or may not be the same as the note generation model 60. The DoPaCo transcript 50 may also be stored on the storage device 130. Alternatively, in some other examples, the generated clinical note 10 and the DoPaCo transcript 50 may be stored on the non-transitory storage 120.

The note generation model 60 may be trained on a collection of historical DoPaCo transcripts, each of which is paired with a corresponding historical clinical note. The note generation model 60 is trained to generate a plurality of pseudo-reference clinical notes 20 based on the DoPaCo transcript 50. The plurality of pseudo-reference clinical notes 20 is "AI generated," i.e., generated or produced by the note generation model 60. The plurality of pseudo-reference clinical notes 20 may include any number of pseudo-reference clinical notes 21, for example, 8 or 16 pseudo-reference clinical notes 21. As discussed above, the production note generation model may or may not be the same as the note generation model 60 that generates the plurality of pseudo-reference clinical notes 20.

In some examples, the note generation model 60 may be invoked to generate the plurality of pseudo-reference clinical notes 20 using a diverse generation approach. That is, the note generation model 60 may employ a plurality of approaches different from each other to generate the plurality of pseudo-reference clinical notes 20. Examples of such approaches include beam search, top-k sampling, top-p sampling, diverse beam search, and so forth. As a result, in some examples, the pseudo-reference clinical notes 21 may be different from each other. As will be described later, the diverse generation approach may improve evaluation of the generated clinical note 10. The note generation model 60 may typically be different from the production note generation model.

The machine learning model 62 may be trained on natural-language understanding tasks such as Natural Language Inference (NLI), which is also known as Textual Entailment. The machine learning model 62, when presented with a pair of sentences or content units, may classify the relationship between the meanings of the pair of sentences or content units by picking one of {entailment, neutral, contradiction}. Therefore, the machine learning model 62 may be used to judge the degree to which two clinical notes agree on any given piece of content, by seeing if one follows from the other (entailment) or contradicts the other.

The machine learning model 62 may be trained to automatically decompose data into finer to a finer level of granularity. The machine learning model 62 may be further trained on health and biomedical data, such as that defined in the Unified Medical Language System (UMLS). In some examples, the machine learning model 62 may also be calibrated/fine-tuned using deidentified historical clinical notes of patients.

A training dataset for the machine learning model 62 may be created by annotating sections of the historical clinical notes with content units, which may be done by medical professionals. Such content units may be referred to as "positive examples." Preferably, data augmentation may be employed to automatically create synthetic negative examples. Specifically, each of the historical clinical note may be paired with a plurality of other historical notes having lowest ROUGE-1 F1 score to create synthetic negative examples. A medical concept engine may also be incorporated into the data augmentation process to exclude matching negatives examples if detected medical concepts between a pair of two historical clinical notes are deemed to be too similar, and to replace medical entities to create new negatives (e.g., swapping medication names and/or doses, etc.).

In some embodiments, the non-transitory storage 120 may further store a knowledge graph 70 of clinical data associated with the patient. The knowledge graph 70 may be generated based on historical clinical data of the patient. For example, the knowledge graph 70 may be generated and updated based on an EHR that stores the historical clinical data of the patient.

As will be discussed in greater detail below, the instructions 65, when executed by the processor 110, may cause the processor 110 to receive the generated clinical note 10 along with the DoPaCo transcript 50, provide the DoPaCo transcript 50 to the note generation model 60 to generate the plurality of pseudo-reference clinical notes 20 via the note generation model 60, evaluate a quality of the generated clinical note 10 based on the plurality of pseudo-reference clinical notes 20, and output the generated clinical note 10 via the output device 140 based on the evaluated quality thereof. The knowledge graph 70 may also be used by the machine learning model 62 for evaluating the generated clinical note 10.

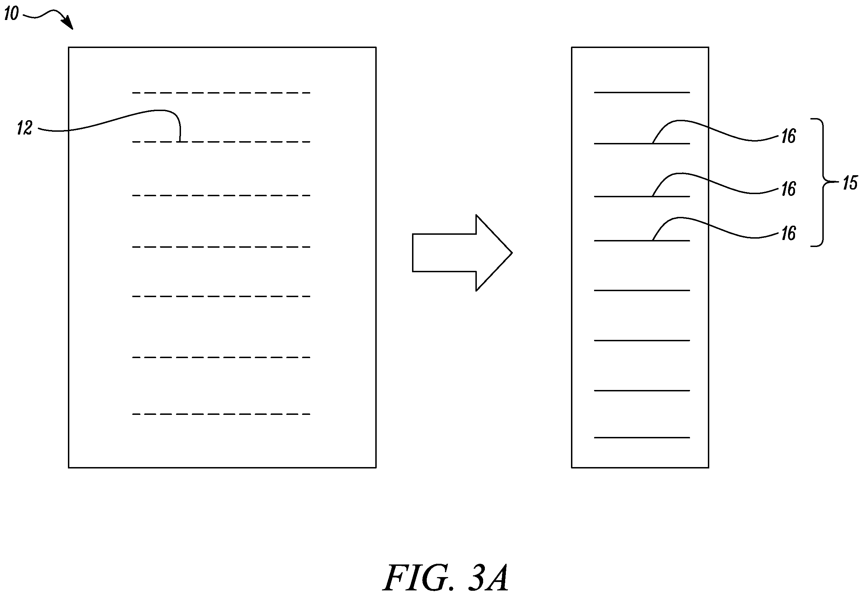

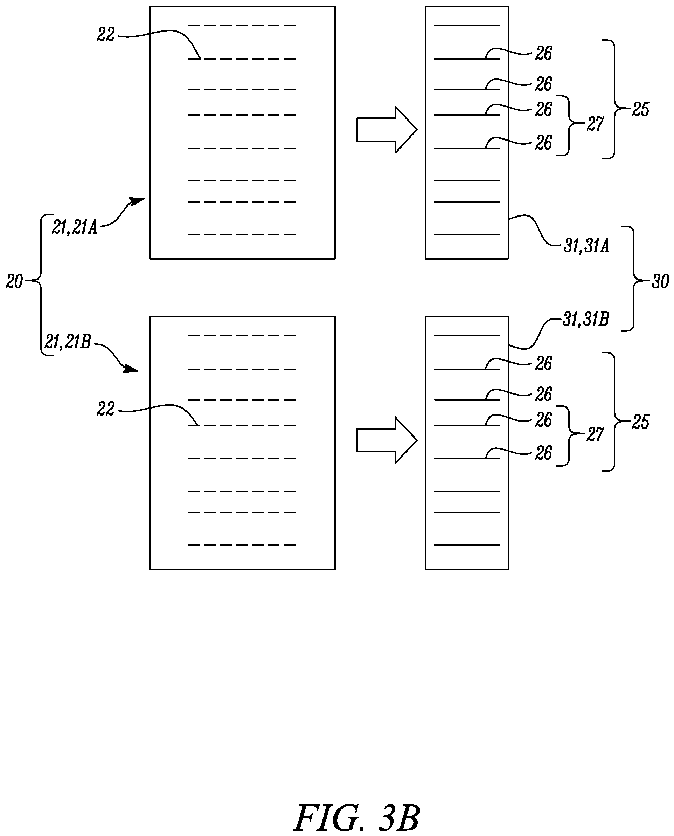

FIG. 3A illustrates a schematic diagram of the generated clinical note 10 produced by the production note generation model, and FIG. 3B illustrates a schematic diagram of two pseudo-reference clinical notes 21 from the plurality of pseudo-reference clinical notes 20 generated by the note generation model 60.

Referring to FIGS. 2 and 3A, the generated clinical note 10 may include clinical data 12 derived from the DoPaCo transcript 50 by the production note generation model. The clinical data 12 may be in the form of text (e.g., phrases, sentences, paragraphs, and so forth). A quality of the generated clinical note 10 may correlate with an accuracy of the clinical data 12, and in some cases, also a formatting of the clinical data 12 in the generated clinical note 10. Thus, the quality of the generated clinical note 10 may depend upon the production note generation model and a quality of the DoPaCo transcript 50.

As will be described in greater detail below, the generated clinical note 10 may be evaluated using at least one evaluation metric. The at least one evaluation metric may include metrics such as ROUGE-N (e.g., ROUGE-1, ROUGE-2, ROUGE-3, ROUGE-4) precision, ROUGE-N recall, ROUGE-N F1, ROUGE-L precision, ROUGE-L recall, ROUGE-L F1, clinical concept precision, clinical concept coverage/recall, Levenshtein distance, and Natural Language Inference (NLI) based metrics, such as content factuality and content coverage. In some examples, the generated clinical note 10 may be evaluated using a plurality of the aforementioned metrics.

For each evaluation metric, the machine learning model 62 may decompose the clinical data 12 of the generated clinical note 10 into a plurality of generated units 15. In other words, for each evaluation metric, the machine learning model 62 may extract the plurality of generated units 15 from the generated clinical note 10.

In some examples, for one evaluation metric, the plurality of generated units 15 may include a plurality of clinical concepts. For the one evaluation metric, each generated unit 16 from the plurality of generated units 15 may correspond to a respective clinical concept from the plurality of clinical concepts. This may be accomplished using clinical concept extraction by the machine learning model 62.

In some examples, for one evaluation metric, the plurality of generated units 15 may include a plurality of semantic concept units (SCUs). For the one evaluation metric, each generated unit 16 from the plurality of generated units 15 may correspond to a respective SCU from the plurality of SCUs. SCUs may refer to units of data obtained by decomposing data to a finer level of granularity (e.g., from paragraphs to sentences, from sentences to clauses/phrases, etc.). As an example, the sentence "His weight went up 6 pounds and he reports his diet is not good" may be decomposed into the following SCUs: a) "His weight went up 6 lbs"; and b) "He reports his diet is not good." Such automatic decomposition of the clinical data 12 into the plurality of SCUs may be performed by the machine learning model 62. In some examples, a sequence-to-sequence model (e.g., BART) may be trained and employed to perform this task of automatically decomposing sentences/paragraphs into the plurality of SCUs.

Referring to FIGS. 2 and 3B, each pseudo-reference clinical note 21 may include clinical data 22 derived from the DoPaCo transcript 50 by the machine learning model 62. The clinical data 22 may be in the form of text (e.g., phrases, sentences, paragraphs, and so forth).

For each evaluation metric, the machine learning model 62 may decompose the clinical data 22 of each pseudo-reference clinical note 21 into a plurality of pseudo units 25. In other words, for each evaluation metric, the machine learning model 62 may extract the plurality of pseudo units 25 from each pseudo-reference clinical note 21.

Specifically, the machine learning model 62 may extract a plurality of sets of pseudo units 30 from the plurality of pseudo-reference clinical notes 20. Each set of pseudo units 31 from the plurality of sets of pseudo units 30 is associated with a respective pseudo-reference clinical note 21 from the plurality of pseudo-reference clinical notes 20. Each set of pseudo units 31 includes the plurality of pseudo units 25. For example, as shown in FIG. 3B, a first set of pseudo units 31A from the plurality of sets of pseudo units 30 may be associated with a first pseudo-reference clinical note 21A from the plurality of pseudo-reference clinical notes 20. That is, the plurality of pseudo units 25 of the first set of pseudo units 31A may be extracted based on the clinical data 22 of the first pseudo-reference clinical note 21A. Further, a second set of pseudo units 31B from the plurality of sets of pseudo units 30 may be associated with a second pseudo-reference clinical note 21B from the plurality of pseudo-reference clinical notes 20. That is, the plurality of pseudo units 25 of the second set of pseudo units 31B may be extracted based on the clinical data 22 of the second pseudo-reference clinical note 21B.

In some examples, for one evaluation metric, the plurality of pseudo units 25 of each set of pseudo units 31 may include a plurality of pseudo clinical concepts. For the one evaluation metric, each pseudo unit 26 from the plurality of pseudo units 25 may correspond to a respective pseudo clinical concept from the plurality of pseudo clinical concepts.

In some examples, for one evaluation metric, the plurality of pseudo units 25 of each set of pseudo units 31 may include a plurality of pseudo SCUs. For the one evaluation metric, each pseudo unit 26 from the plurality of pseudo units 25 may correspond to a respective pseudo SCU from the plurality of pseudo SCUs.

Referring to FIGS. 2, 3A, and 3B, the plurality of generated units 15 and the plurality of pseudo units 25 may be different for different evaluation metrics. For example, for one evaluation metric, the plurality of generated units 15 and the plurality of pseudo units 25 of each set of pseudo units 31 may be a plurality of clinical concepts, and for another evaluation metric, the plurality of generated units 15 and the plurality of pseudo units 25 of each set of pseudo units 31 may be a plurality of SCUs. For evaluation of the generated clinical note 10 based on one evaluation metric, the plurality of generated units 15 of the generated clinical note 10 may be compared with the plurality of pseudo units 25 of each set of pseudo units 31 from the plurality of sets of pseudo units 30 corresponding to the one evaluation metric.

The instructions 65, when executed by the processor 110, cause the processor 110 to receive the generated clinical note 10 associated with the patient and the DoPaCo transcript 50 based on which the generated clinical note 10 is generated. As discussed above, the generated clinical note 10 is generated or produced by the production note generation model based on the DoPaCo transcript 50.

The instructions 65 further cause the processor 110 to provide the DoPaCo transcript 50 to the note generation model 60. As discussed above, the note generation model 60 is trained to generate the plurality of pseudo-reference clinical notes 20 based on the DoPaCo transcript 50.

The instructions 65 further cause the processor 110 to generate, via the note generation model 60, the plurality of pseudo-reference clinical notes 20 based on the DoPaCo transcript 50. As discussed above, in some implementations, the note generation model 60 may generate the plurality of pseudo-reference clinical notes 20 using a diverse generation approach.

The instructions 65 further cause the processor 110 to select at least one evaluation metric for evaluating the generated clinical note 10. In some examples, the at least one evaluation metric may include a plurality of evaluation metrics. Using the plurality of evaluation metrics to evaluate the generated clinical note 10 may reduce metric bias (i.e., prediction of high metric scores for production clinical notes) which may otherwise arise from using a single evaluation metric for evaluating the generated clinical note 10. Further, as each evaluation metric may be related to an aspect of errors that contribute to extra effort/time for correcting the generated clinical note 10, it may be beneficial to use the plurality of evaluation metrics to evaluate the generated clinical note 10. Specifically, some of the aspects of errors may be more important or less important to different medical professionals. The plurality of evaluation metrics may be weighted differently for each medical professional, depending on their preferences. Thus, in such cases, "the at least one evaluation metric" may be interchangeably considered as "the plurality of evaluation metrics."

The instructions 65 further cause the processor 110 to extract, via the machine learning model 62, for each evaluation metric, the plurality of generated units 15 from the generated clinical note 10.

The instructions 65 further cause the processor 110 to extract, via the machine learning model 62, for each evaluation metric, the plurality of sets of pseudo units 30 from the plurality of pseudo-reference clinical notes 20. Each set of pseudo units 31 from the plurality of sets of pseudo units 30 is associated with the respective pseudo-reference clinical note 21 from the plurality of pseudo-reference clinical notes 20. Each set of pseudo units 31 includes the plurality of pseudo units 25.

The instructions 65 further cause the processor 110 to compare, via the machine learning model 62, for each evaluation metric, the plurality of generated units 15 and the plurality of pseudo units 25 of each set of pseudo units 31 corresponding to the evaluation metric. For example, for a clinical concept precision metric, the plurality of generated clinical concepts of the generated clinical note 10 and the plurality of pseudo clinical concepts of each set of pseudo units 31 may be compared. As another example, for a content factuality metric, the plurality of generated SCUs of the generated clinical note 10 and the plurality of pseudo SCUs of each set of pseudo units 31 may be compared.

Referring now to FIGS. 2-4, the instructions 65 further cause the processor 110 to calculate, for each evaluation metric, a plurality of sub-scores 35 for the generated clinical note 10 based on the comparison of the plurality of generated units 15 and the plurality of pseudo units 25 of each set of pseudo units 31 corresponding to the evaluation metric. Each sub-score 36 from the plurality of sub-scores 35 corresponding to a respective evaluation metric is associated with a respective pseudo-reference clinical note 21.

Each sub-score 36 may be represented in terms of a fraction. Each sub-score 36 may range from 0 to 1, where 0 is the lowest score and 1 is the highest score. For each evaluation metric, each sub-score 36 from the plurality of sub-scores 35 may indicate similarity/likeness of the clinical data 12 of the generated clinical note 10 and the clinical data 22 of the respective pseudo-reference clinical note 21. It may be noted that different sub-scores 36 may be obtained for different evaluation metrics. For example, the sub-score 36 associated with the respective pseudo-reference clinical note 21 and calculated based on ROUGE-1 precision metric may be different from the sub-score 36 associated with the respective pseudo-reference clinical note 21 and calculated based on ROUGE-1 recall metric. As another example, the sub-score 36 associated with the respective pseudo-reference clinical note 21 and calculated based on clinical concept precision metric may be different from the sub-score 36 associated with the respective pseudo-reference clinical note 21 and calculated based on content factuality metric.

In cases where the plurality of evaluation metrics is used for evaluation, the processor 110 may calculate a plurality of sets of sub-scores 37 corresponding to the plurality of evaluation metrics. Each set of sub-scores 38 from the plurality of sets of sub-scores 37 may correspond to a respective evaluation metric from the plurality of evaluation metrics. Each set of sub-scores 38 may include the plurality of sub-scores 35.

For example, a first set of sub-scores 38A from the plurality of sets of sub-scores 37 may correspond to a first evaluation metric from the plurality of evaluation metrics. A first sub-score 36A1 for the generated clinical note 10 for the first evaluation metric may be associated with (i.e., be in respect with) the first pseudo-reference clinical note 21A, a second sub-score 36B1 for the generated clinical note 10 for the first evaluation metric may be associated with (i.e., be in respect with) the second pseudo-reference clinical note 21B, and so forth. Further, a second set of sub-scores 38B from the plurality of sets of sub-scores 37 may correspond to a second evaluation metric from the plurality of evaluation metrics. A first sub-score 36A2 for the generated clinical note 10 for the second evaluation metric may be associated with (i.e., be in respect with) the first pseudo-reference clinical note 21A, a second sub-score 36B2 of the generated clinical note 10 for the second evaluation metric may be associated with (i.e., be in respect with) the second pseudo-reference clinical note 21B, and so forth.

In some embodiments, the at least one evaluation metric may include a clinical concept precision metric. Corresponding to the clinical concept precision metric, the plurality of generated units 15 may include a plurality of generated clinical concepts and the plurality of pseudo units 25 of each set of pseudo units 31 may include a plurality of pseudo clinical concepts. For the clinical concept precision metric, each sub-score 36 associated with the respective pseudo-reference clinical note 21 may be calculated based on a ratio of (i) a number of the plurality of generated clinical concepts that match with the plurality of pseudo clinical concepts of the set of pseudo units 31 corresponding to the respective pseudo-reference clinical note 21 to (ii) a total number of the plurality of generated clinical concepts of the generated clinical note 10.

For example, if the total number of the plurality of generated clinical concepts is 10, and 5 of the plurality of generated clinical concepts match with the plurality of pseudo clinical concepts of the set of pseudo units 31 corresponding to the respective pseudo-reference clinical note 21, then, for the clinical concept precision metric, the sub-score 36 associated with the respective pseudo-reference clinical note 21 may be based on the ratio 5/10 or 0.5.

In some embodiments, the at least one evaluation metric may include a clinical concept coverage metric. Corresponding to the clinical concept coverage metric, the plurality of generated units 15 may include the plurality of generated clinical concepts and the plurality of pseudo units 25 of each set of pseudo units 31 may include the plurality of pseudo clinical concepts. For the clinical concept coverage metric, each sub-score 36 associated with the respective pseudo-reference clinical note 21 may be calculated based on a ratio of (i) a number of the plurality of pseudo clinical concepts of the set of pseudo units 31 corresponding to the respective pseudo-reference clinical note 21 that match with the plurality of generated clinical concepts of the generated clinical note 10 to (ii) a total number of the plurality of pseudo clinical concepts of the set of pseudo units 31 corresponding to the respective pseudo-reference clinical note 21.

For example, if the total number of the plurality of pseudo clinical concepts of the set of pseudo units 31 corresponding to the respective pseudo-reference clinical note 21 is 10, and 5 of the plurality of pseudo clinical concepts of the set of pseudo units 31 corresponding to the respective pseudo-reference clinical note 21 match with the plurality of generated clinical concepts of the generated clinical note 10, then, for the clinical concept coverage metric, the sub-score 36 associated with the respective pseudo-reference clinical note 21 may be based on the ratio 5/10 or 0.5.

In some embodiments, the at least one evaluation metric may include a content factuality metric. Corresponding to the content factuality metric, the plurality of generated units 15 may include a plurality of generated SCUs and the plurality of pseudo units 25 of each set of pseudo units 31 may include a plurality of pseudo SCUs. For the content factuality metric, each sub-score 36 associated with the respective pseudo-reference clinical note 21 may be calculated by aggregating a plurality of generated entailment probabilities corresponding to the plurality of generated SCUs. Each generated entailment probability from the plurality of generated entailment probabilities may be associated with a respective generated SCU and correspond to a maximum entailment probability of the respective generated SCU with respect to the plurality of pseudo SCUs of the respective pseudo-reference clinical note 21.

For example, if the respective pseudo-reference clinical note 21 has three pseudo SCUs, and a first generated SCU is 70% entailed by the first pseudo SCU, 90% entailed by the second pseudo SCU, and 10% entailed by the third pseudo SCU, then, the maximum entailment probability (which is 90% in this example) may be an entailment score of the first generated SCU. In other words, each of the three pseudo SCUs may be considered as the "premise" and the first generated SCU may be considered as the "hypothesis" to calculate the maximum entailment probability for the first generated SCU, and subsequently the entailment score of the first generated SCU. Similarly, the entailment scores for all the generated SCUs of the generated clinical note 10 may be calculated and aggregated to determine, for the content factuality metric, the sub-score 36 for the generated clinical note 10 associated with the respective pseudo-reference clinical note 21.

Alternatively, in some embodiments, for the content factuality metric, a "top-k pseudo approach" may be used to determine the sub-score 36. Specifically, for each generated SCU, a plurality of top pseudo SCUs may be determined, where the plurality of top pseudo SCUs includes the pseudo SCUs having maximum entailment probabilities greater than a predetermined entailment threshold with respect to the generated SCU. For each generated SCU, the plurality of top pseudo SCUs may be concatenated to form a "top-k pseudo premise." For the content factuality metric, for each generated SCU, the entailment score of the generated SCU may be determined by considering the generated SCU as the "hypothesis" against the determined top-k pseudo premise. Similarly, the entailment scores for all the generated SCUs may be calculated and aggregated to determine, for the content factuality metric, the sub-score 36 for the generated clinical note 10 associated with the respective pseudo-reference clinical note 21.

In some embodiments, the at least one evaluation metric may include a content coverage metric. Corresponding to the content coverage metric, the plurality of generated units 15 may include the plurality of generated SCUs and the plurality of pseudo units 25 of each set of pseudo units 31 may include a plurality of pseudo SCUs. For the content coverage metric, each sub-score 36 associated with the respective pseudo-reference clinical note 21 may be calculated by aggregating a plurality of pseudo entailment probabilities corresponding to the plurality of pseudo SCUs of the respective pseudo-reference clinical note 21. Each pseudo entailment probability from the plurality of pseudo entailment probabilities may be associated with a respective pseudo SCU and correspond to a maximum entailment probability of the respective pseudo SCU with respect to the plurality of generated SCUs of the generated clinical note 10.

For example, if the generated clinical note 10 has three generated SCUs, and a first pseudo SCU is 50% entailed by the first generated SCU, 80% entailed by the second generated SCU, and 20% entailed by the third generated SCU, then, the maximum entailment probability (which is 80% in this example) may be an entailment score of the first pseudo SCU. In other words, each of the three generated SCUs may be considered as the "premise" and the first pseudo SCU may be considered as the "hypothesis" to calculate the maximum entailment probability for the first pseudo SCU, and subsequently the entailment score of the first pseudo SCU. Similarly, the entailment scores for all the pseudo SCUs of the set of pseudo units 31 corresponding to the respective pseudo-reference clinical note 21 may be calculated and aggregated to determine, for the content coverage metric, the sub-score 36 for the generated clinical note 10 associated with the respective pseudo-reference clinical note 21.

Alternatively, in some embodiments, for the content coverage metric, a "top-k generated approach" may be used to determine the sub-score 36. Specifically, for each pseudo SCU, a plurality of top generated SCUs may be determined, where the plurality of top generated SCUs includes the generated SCUs having maximum entailment probabilities greater than a predetermined entailment threshold with respect to the pseudo SCU. For each pseudo SCU, the plurality of top generated SCUs may be concatenated to form a "top-k generated premise." For the content coverage metric, for each pseudo SCU, the entailment score of the pseudo SCU may be determined by considering the pseudo SCU as the "hypothesis" against the determined top-k generated premise. Similarly, the entailment scores for all the pseudo SCUs may be calculated and aggregated to determine, for the content coverage metric, the sub-score 36 for the generated clinical note 10 associated with the respective pseudo-reference clinical note 21.

Referring to FIGS. 2, 4, and 5A, in some embodiments, the instructions 65 may further cause the processor 110 to receive a plurality of transcript confidence scores 53 corresponding to a plurality of transcript units 51 of the DoPaCo transcript 50. Each transcript confidence score 54 from the plurality of transcript confidence scores 53 may correspond to a respective transcript unit 52 from the plurality of transcript units 51. For example, the plurality of transcript units 51 may include first, second, and third transcript units 52A, 52B, 52C. The first transcript unit 52A may have a first transcript confidence score 54A, the second transcript unit 52B may have a second transcript confidence score 54B, and the third transcript unit 52C may have a third transcript confidence score 54C. The confidence scoring of the plurality of transcript units 51 may be performed by the production note generation model, the machine learning model 62, or an Automatic Speech Recognition (ASR) model.

The instructions 65 may further cause the processor 110 to determine, via the machine learning model 62, for one or more evaluation metrics from the at least one evaluation metric, which of the plurality of generated units 15 correspond to which of the plurality of transcript units 51. For example, the processor 110 may determine, via the machine learning model 62, that a first generated unit 16A corresponds to the first transcript unit 52A, a second generated unit 16B corresponds to the second transcript unit 52B, and a third generated unit 16C corresponds to the third transcript unit 52C.

The instructions 65 may further cause the processor 110 to assign, via the machine learning model 62, for the one or more evaluation metrics, a plurality of transcript weights 55 to the plurality of generated units 15 based on the plurality of transcript confidence scores 53. Each transcript weight 56 from the plurality of transcript weights 55 may be assigned to a corresponding generated unit 16 from the plurality of generated units 15. For example, the processor 110 may assign, via the machine learning model 62, a first transcript weight 56A to the first generated unit 16A, a second transcript weight 56B to the second generated unit 16B, and a third transcript weight 56C to the third generated unit 16C. For the one or more evaluation metrics, the plurality of sub-scores 35 may be calculated further based on the plurality of transcript weights 55.

Referring now to FIGS. 2, 4, and 5B, in some embodiments, the instructions 65 may further cause the processor 110 to assign, for each evaluation metric, a plurality of knowledge weights 71 to the plurality of generated units 15 based on the knowledge graph 70. Each generated unit 16 may be assigned with a corresponding knowledge weight 72 from the plurality of knowledge weights 71. For one or more evaluation metrics from the at least one evaluation metric, the plurality of sub-scores 36 may be calculated further based on the plurality of knowledge weights 71. For example, if the knowledge graph 70 includes evidence of diabetes, the generated units 16 related to diabetes may be assigned a higher weight than those generated units 16 that have missing or contradicting evidence in the knowledge graph 70.

In some embodiments, the instructions 65 may further cause the processor 110 to generate, via the machine learning model 62, a plurality of pseudo knowledge graphs (not shown) corresponding to the plurality of pseudo-reference clinical notes 20. The instructions 65 may further cause the processor 110 to compare the knowledge graph 70 and the plurality of pseudo knowledge graphs. The instructions 65 may further cause the processor 110 to calculate a knowledge metric score based on the comparison of the knowledge graph 70 and the plurality of pseudo knowledge graphs.

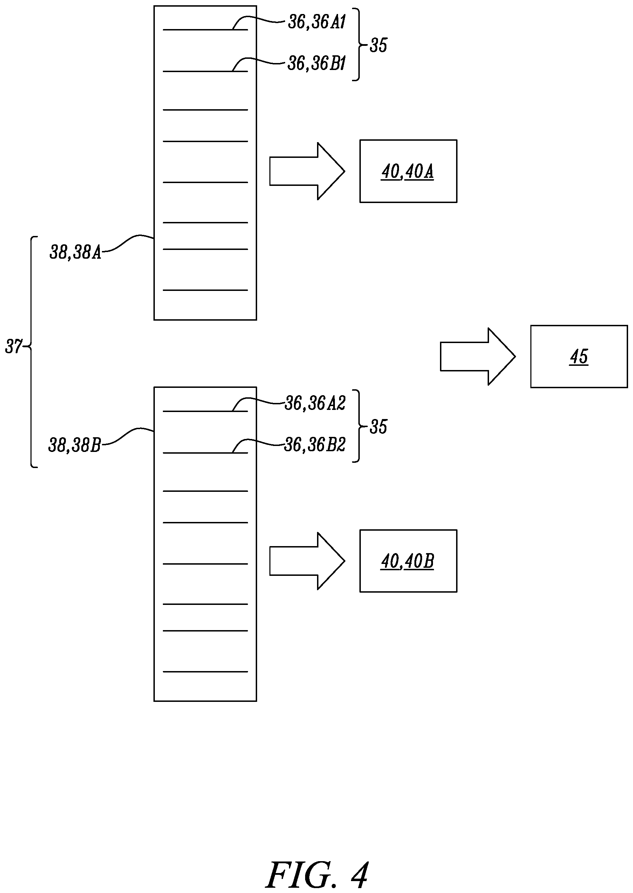

Referring to FIGS. 2 and 4, the instructions 65 further cause the processor 110 to aggregate, for each evaluation metric, the plurality of sub-scores 35 corresponding to the evaluation metric to calculate a metric score 40 corresponding to the evaluation metric.

For example, the first set of sub-scores 38A may correspond to the first evaluation metric, and the second set of sub-scores 38B may correspond to the second evaluation metric. The processor 110 may aggregate the plurality of sub-scores 35 of the first set of sub-scores 38A to determine a first metric score 40A corresponding to the first evaluation metric, and the plurality of sub-scores 35 of the second set of sub-scores 38B to determine a second metric score 40B corresponding to the second evaluation metric.

Referring to FIGS. 2, 3A, and 4, the instructions 65 further cause the processor 110 to aggregate, via the machine learning model 62, the metric score 40 corresponding to each of the at least one evaluation metric to predict a quality score 45 of the generated clinical note 10. For example, the processor 110 may aggregate, via the machine learning model 62, the first metric score 40A corresponding to the first evaluation metric and the second metric score 40B corresponding to the second evaluation metric to determine the quality score 45 of the generated clinical note 10. It may be noted that the aggregation of the metric scores 40 may be weighted. For example, the metric scores 40 corresponding to the content factuality metric and the content coverage metric may be weighted higher than others of the at least one evaluation metric.

The instructions 65 further cause the processor 110 to output, via the output device 140, the generated clinical note 10 if the quality score 45 is greater than or equal to a predetermined acceptable threshold. The predetermined acceptable threshold may be set based on a desired quality of the generated clinical note 10. As an example, the quality score 45 may be represented as a percentage. In such cases, the predetermined acceptable threshold may be, for example, 50%, 60%, 70%, etc.

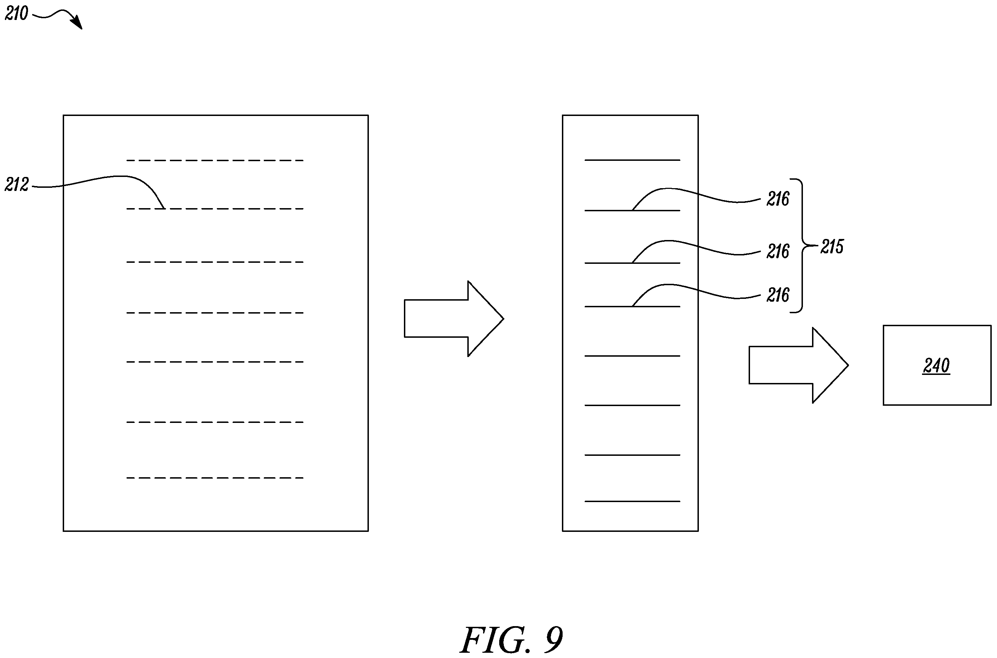

The instructions 65 further cause the processor 110 to output, via the output device 140, a request to manually draft a drafted clinical note 210 (shown in FIG. 9) based on the DoPaCo transcript 50 if the quality score 45 is less than the predetermined acceptable threshold. In some cases, manually drafting the drafted clinical note 210 may be easier and/or quicker than correcting the generated clinical note 10 if the quality score 45 is less than the predetermined acceptable threshold.

The system 101 may employ the machine learning model 62 to predict the quality score 45 of the generated clinical note 10, which may correlate with an effort required to improve/correct the generated clinical note 10 to an acceptable quality level. Specifically, the note generation model 60 generates the plurality of pseudo-reference clinical notes 20, which is utilized by the machine learning model 62 as missing human-written notes (since there are no human-written notes available in a production environment) corresponding to the DoPaCo transcript 50.

Further, the system 101 may evaluate the generated clinical note 10 based on a plurality of evaluation metrics. Each evaluation metric may be related to an aspect of errors that contribute to extra effort/time for correcting the generated clinical note 10. Therefore, evaluating the generated clinical note 10 based on the plurality of evaluation metrics may encompass various aspects that contribute to extra effort/time for correcting the generated clinical note 10 and reduce metric bias of each individual evaluation metric.

For example, the metric score 40 corresponding to the clinical concept precision metric and the metric score 40 corresponding to the clinical concept coverage/recall metric may be combined to predict an amount of effort required by a medical professional (e.g., scribe or physician) to fix the incorrect clinical concepts present in the generated clinical note 10 and add clinical concepts missing from the generated clinical note 10.

The system 101 may also provide explanations for the predicted quality score 45 and hints for how the generated clinical note 10 can be improved, for example, by highlighting portions of the generated clinical note 10 that are predicted to be of low quality, and adding missing data to the generated clinical note 10.

Referring to FIGS. 2, 3A, 3B, and 6, in some embodiments, the instructions 65 further cause the processor 110 to calculate, for one evaluation metric from the at least one evaluation metric, a plurality of unit scores 17 for the plurality of generated units 15 of the generated clinical note 10 based on the comparison of the plurality of generated units 15 and the plurality of pseudo units 25 of each set of pseudo units 31 corresponding to the one evaluation metric. Each unit score 18 from the plurality of unit scores 17 may correspond to a respective generated unit 16 from the plurality of generated units 15. For example, the processor 110 may calculate, for the clinical concept precision metric, the plurality of unit scores 17 for the plurality of generated units 15. Each unit score 18 may be represented in terms of a fraction. Each unit score 18 may range from 0 to 1, where 0 is the lowest score and 1 is the highest score.

The instructions 65 may further cause the processor 110 to determine which of the plurality of generated units 15 have the unit scores 18 less than a predetermined unit threshold. The predetermined unit threshold may be set based on a desired acceptable quality of each of the plurality of generated units 15. The instructions 65 may further cause the processor 110 to determine one or more portions 13 of the generated clinical note 10 corresponding to the plurality of generated units 15 having the unit scores 18 less than the predetermined unit threshold.

The instructions 65 may further cause the processor 110 to highlight the one or more portions 13 of the generated clinical note 10 corresponding to the plurality of generated units 15 having the unit scores 18 less than the predetermined unit threshold prior to outputting the generated clinical note 10.

Advantageously, the highlighted portions (i.e., the one or more portions 13) of the generated clinical note 10 may help the medical professional in quickly identifying potential errors in the generated clinical note 10.

Referring to FIGS. 2, 3A, 3B, and 4, in some embodiments, the instructions 65 further cause the processor 110 to compare, via the machine learning model 62, for each evaluation metric, the plurality of pseudo units 25 of the plurality of sets of pseudo units 30 with each other. For example, the plurality of pseudo units 25 of the first set of pseudo units 31A may be compared with the plurality of pseudo units 25 of the second set of pseudo units 31B.

The instructions 65 may further cause the processor 110 to determine, for each evaluation metric, a plurality of missing pseudo units 27 (shown in FIG. 3B) from the plurality of pseudo units 25. The plurality of missing pseudo units 27 may include the pseudo units 26 that are missing equivalent generated units 16 from the plurality of generated units 15 and that repeat among the plurality of sets of pseudo units 30 based on the comparison of the plurality of pseudo units 25 of the plurality of sets of pseudo units 30 with each other.

The instructions 65 may further cause the processor 110 to add the plurality of missing pseudo units 27 to the generated clinical note 10 before outputting the generated clinical note 10 if the quality score 45 is greater than or equal to the predetermined acceptable threshold. In some examples, the plurality of missing pseudo units 27 may be added to the generated clinical note 10 in the form of sentences, clauses, phrases, etc. in the clinical data 12 to improve a coherence of the generated clinical note 10.

As a result, the generated clinical note 10 may include missing clinical data (i.e., the plurality of missing pseudo units 27) when outputted via the output device 140. Advantageously, this may improve the quality of the generated clinical note 10 when displayed to the medical professional. In some cases, the plurality of missing pseudo units 27 added to the generated clinical note 10 may be highlighted (or formatted differently from the original clinical data 12 of the generated clinical note 10) to indicate that the plurality of missing pseudo units 27 has been added to the generated clinical note 10. The medical professional may therefore quickly identify and review the plurality of missing pseudo units 27 added to the generated clinical note 10.

Moreover, the system 101 may employ quality prediction in a section-by-section manner. That is, the plurality of generated units 15 of one section of the generated clinical note 10 may be compared with the plurality of pseudo units 25 of each of the plurality of pseudo-reference clinical notes 20 of the same section, rather than comparing the generated clinical note 10 and the plurality of pseudo-reference clinical notes 20 in their entirety.

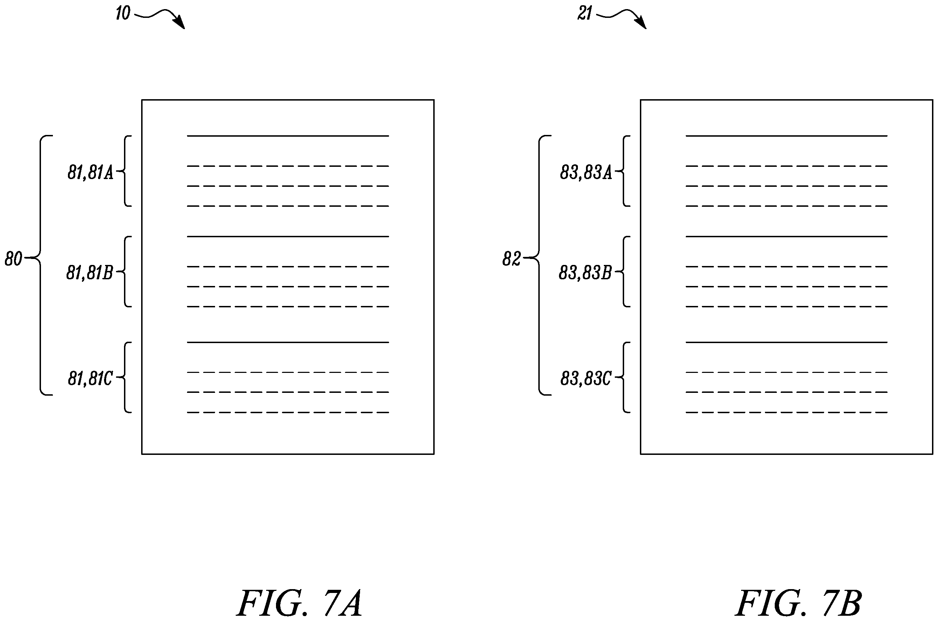

Referring to FIGS. 2, 7A, and 7B, in some embodiments, the generated clinical note 10 may include a plurality of generated note sections 80 and each of the plurality of pseudo-reference clinical notes 20 may include a plurality of pseudo note sections 82 corresponding to the plurality of generated note sections 80. Each pseudo note section 83 from the plurality of pseudo note sections 82 may correspond to a respective generated note section 81 from the plurality of generated note sections 80. The plurality of generated note sections 80 and the plurality of pseudo note sections 82 may include, for example, Chief Complaint (CC), History of Present Illness (HPI), Physical Examination (PE), Assessment and Plan (A&P), etc.

As an example, the plurality of generated note sections 80 may include a first generated note section 81A, a second generated note section 81B, and a third generated note section 81C. Further, each of the plurality of pseudo-reference clinical notes 20 may include a first pseudo note section 83A corresponding to the first generated note section 81A, a second pseudo note section 83B corresponding to the second generated note section 81B, and a third pseudo note section 83C corresponding to the third generated note section 81C.

Referring to FIGS. 2, 3A, 3B, 7A, and 7B, the instructions 65 may further cause the processor 110 to compare, via the machine learning model 62, for each evaluation metric, the plurality of generated units 15 of each generated note section 81 and the plurality of pseudo units 25 of a respective pseudo note section 83 corresponding to the generated note section 81 of each set of pseudo units 31. For example, the plurality of generated units 15 of the first generated note section 81A may be compared with the plurality of pseudo units 25 of the first pseudo note section 83A of each pseudo-reference clinical note 21, the plurality of generated units 15 of the second generated note section 81B may be compared with the plurality of pseudo units 25 of the second pseudo note section 83B of each pseudo-reference clinical note 21, and the plurality of generated units 15 of the third generated note section 81C may be compared with the plurality of pseudo units 25 of the third pseudo note section 83C of each pseudo-reference clinical note 21.

Referring now to FIGS. 2, 7A, and 8, the instructions 65 may further cause the processor 110 to calculate, for each evaluation metric, a plurality of section sub-scores 84 for each generated note section 81 of the generated clinical note 10 based on the comparison of the plurality of generated units 15 of each generated note section 81 and the plurality of pseudo units 25 of the respective pseudo note section 83 of each set of pseudo units 31. Each section sub-score 85 from the plurality of section sub-scores 84 corresponding to a respective evaluation metric is associated with a respective pseudo-reference clinical note 21. Each section sub-score 85 may be represented in terms of a fraction. Each section sub-score 85 may range from 0 to 1, where 0 is the lowest score and 1 is the highest score.

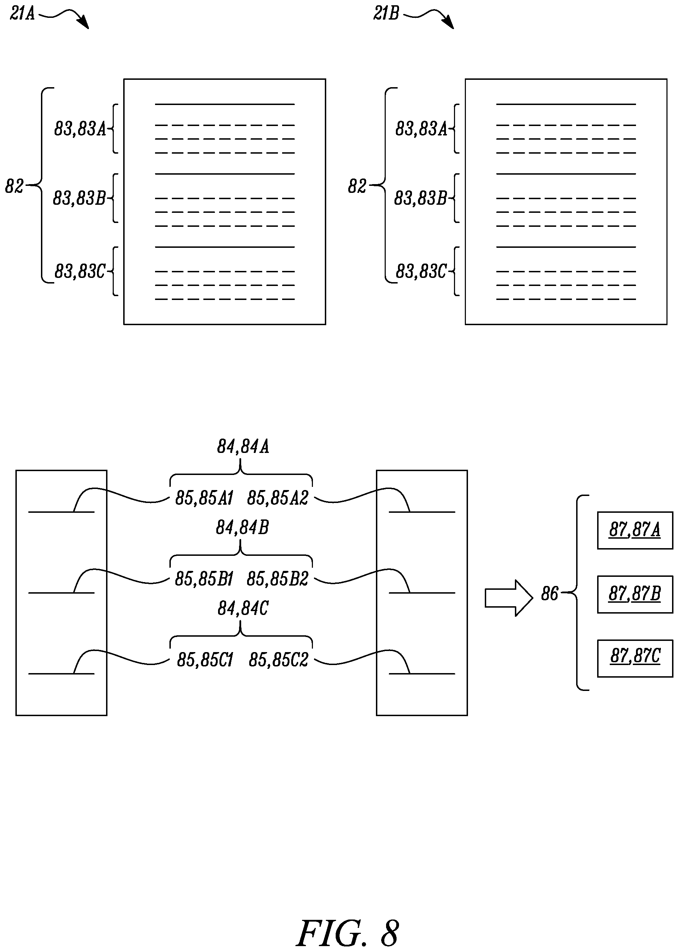

As an example, the plurality of section sub-scores 84 calculated for one evaluation metric will be explained with respect to the first pseudo-reference clinical note 21A and the second pseudo-reference clinical note 21B shown in FIG. 8. For one evaluation metric, the processor 110 may calculate a first plurality of section sub-scores 84A for the first generated note section 81A. The first plurality of section sub-scores 84A may include a first section sub-score 85A1 associated with the first pseudo-reference clinical note 21A and a second section sub-score 85A2 associated with the second pseudo-reference clinical note 21B. The first section sub-score 85A1 may be calculated based on the comparison of the plurality of generated units 15 of the first generated note section 81A and the plurality of pseudo units 25 of the first pseudo note section 83A of the first pseudo-reference clinical note 21A. The second section sub-score 85A2 may be calculated based on the comparison of the plurality of generated units 15 of the first generated note section 81A and the plurality of pseudo units 25 of the first pseudo note section 83A of the second pseudo-reference clinical note 21B.

Further, for the one evaluation metric, the processor 110 may further calculate a second plurality of section sub-scores 84B for the second generated note section 81B. The second plurality of section sub-scores 84B may include a first section sub-score 85B1 associated with the first pseudo-reference clinical note 21A and a second section sub-score 85B2 associated with the second pseudo-reference clinical note 21B. The first section sub-score 85B1 may be calculated based on the comparison of the plurality of generated units 15 of the second generated note section 81B and the plurality of pseudo units 25 of the second pseudo note section 83B of the first pseudo-reference clinical note 21A. The second section sub-score 85B2 may be calculated based on the comparison of the plurality of generated units 15 of the second generated note section 81B and the plurality of pseudo units 25 of the second pseudo note section 83B of the second pseudo-reference clinical note 21B.

In a similar manner, for the one evaluation metric, the processor 110 may calculate a third plurality of section sub-scores 84C for the third generated note section 81C. The third plurality of section sub-scores 84C may include a first section sub-score 85C1 associated with the first pseudo-reference clinical note 21A and a second section sub-score 85C2 associated with the second-reference pseudo-reference clinical note 21B.

The instructions 65 may further cause the processor 110 to aggregate, for each evaluation metric, the plurality of section sub-scores 84 for each generated note section 81 to calculate a plurality of section scores 86 for the plurality of generated note sections 81. Each generated note section 81 has a corresponding section score 87 from the plurality of section scores 86. For example, the first plurality of section sub-scores 84A may be aggregated to calculate a first section score 87A for the first generated note section 81A, the second plurality of section sub-scores 84B may be aggregated to calculate a second section score 87B for the second generated note section 81B, and the third plurality of section sub-scores 84C may be aggregated to calculate a third section score 87C for the third generated note section 81C.

Referring to FIGS. 2, 4, and 8, in some embodiments, for each evaluation metric, the plurality of section scores 86 may be aggregated with the plurality of sub-scores 35 to calculate the metric score 40. In some embodiments, the instructions 65 may further cause the processor 110 to modify at least one generated note section 81 from the plurality of generated note sections 80 of the generated clinical note 10 based on the section score 87 of the at least one generated note section 81 prior to outputting the generated clinical note 10. Modifying the at least one generated note section 81 may include removing the at least one generated note section 81 from the generated clinical note 10 if the section score 87 is less than a predetermined acceptable section threshold, and highlighting the at least one generated note section 81 of the generated clinical note 10 if the section score is greater than or equal to the predetermined acceptable section threshold.

Advantageously, if one generated note section 81 has a very low quality (less than the predetermined acceptable section threshold) but rest of the plurality of generated note sections 80 have acceptable quality (greater than the predetermined acceptable section threshold), the one generated note section 81 may be removed from the generated clinical note 10, so that the one generated note section 81 may be manually written by the medical professional (e.g., a request to manually draft the one generated note section 81 may be output). Further, if one generated note section 81 has an acceptable quality (greater than the predetermined acceptable section threshold), but not a very high quality, then the one generated note section 81 may be highlighted for review.

Referring to FIGS. 2, 4, and 9, as discussed above, the instructions 65 further cause the processor 110 to output, via the output device 140, the request to manually draft the drafted clinical note 210 based on the DoPaCo transcript if the quality score 45 is less than the predetermined acceptable threshold.

In some embodiments, the instructions 65 may further cause the processor 110 to receive the drafted clinical note 210. The drafted clinical note 210 may include clinical data 212 derived from the DoPaCo transcript 50 by the medical professional. The clinical data 212 may be in the form of text (e.g., phrases, sentences, paragraphs, and so forth). The clinical data 212 may be written by the medical professional from scratch using, for example, the input device 150.

The instructions 65 may further cause the processor 110 to extract, via the machine learning model 62, for each evaluation metric, a plurality of drafted units 215 from the drafted clinical note 210. In other words, for each evaluation metric, the machine learning model 62 may decompose the clinical data 212 of the drafted clinical note 210 into the plurality of drafted units 215. Each drafted unit 216 from the plurality of drafted units 215 may correspond to a respective evaluation metric from the at least one evaluation metric. In some embodiments, for one evaluation metric, each drafted unit 216 may be a clinical concept. In some embodiments, for one evaluation metric, each drafted unit 216 may be a SCU.

Referring to FIGS. 2, 3A, and 9, the instructions 65 may further cause the processor 110 to compare, via the machine learning model 62, for each evaluation metric, the plurality of generated units 15 and the plurality of drafted units 215 corresponding to the evaluation metric.

The instructions 65 may further cause the processor 110 to calculate, for each evaluation metric, a draft reference metric score 240 based on the comparison of the plurality of generated units 15 and the plurality of drafted units 215 corresponding to the evaluation metric. The draft reference metric score 240 may be represented in terms of a fraction. The draft reference metric score 240 may range from 0 to 1, where 0 is the lowest score and 1 is the highest score.

Referring to FIGS. 2, 4, 9, and 10, in case of a plurality of evaluation metrics, the processor 110 may calculate a plurality of draft reference metric scores 239 corresponding to the plurality of evaluation metrics. For example, the plurality of draft reference metric scores 239 may include a first draft reference metric score 240A corresponding to a first evaluation metric, a second draft reference metric score 240B corresponding to a second evaluation metric, a third draft reference metric score 240C corresponding to a third evaluation metric, and so forth.

The instructions 65 may further cause the processor 110 to aggregate, via the machine learning model 62, the draft reference metric score 240 corresponding to each of the at least one evaluation metric to determine a draft reference quality score 245 of the generated clinical note 10. For example, the processor 110 may aggregate the plurality of draft reference metric scores 239, or more specifically, the first, second, and third draft reference metric scores 240A, 240B, 240C to determine the draft reference quality score 245 of the generated clinical note 10. The aggregation of the plurality of draft reference metric scores 239 may be weighted.

The instructions 65 may further cause the processor 110 to update the machine learning model 62 based on the quality score 45 and the draft reference quality score 245. Specifically, the processor 110 may update the machine learning model 62 (e.g., re-train the machine learning model 62, calibrate or tweak the machine learning model 62, change aggregation weights of the plurality of evaluation metrics, and so forth) to reduce a difference between the quality score 45 and the draft reference quality score 245. One advantage of using the drafted clinical note 210 for updating the machine learning model 62 may be that since the drafted clinical note 210 is written from scratch by the medical practitioner, the draft reference quality score 245 may be free from metric bias (i.e., prediction of high metric scores for production clinical notes), which may otherwise arise if a clinical note is produced by amending the generated clinical note 10.

The instructions 65 may further cause the processor 110 to output the updated machine learning model 62 to at least one of the storage device 130 and the non-transitory storage 120. This may allow the updated (and improved) machine learning model 62 to be used to predict the quality scores 45 of subsequent generated clinical notes.

Referring to FIGS. 2 and 4, as discussed above, the instructions 65 further cause the processor 110 to output, via the output device 140, the generated clinical note 10 (which may be modified before being output according to the present disclosure) if the quality score 45 is greater than or equal to the predetermined acceptable threshold.

Referring to FIGS. 2, 4, and 11, the instructions 65 may further cause the processor 110 to receive an amended clinical note 310. The amended clinical note 310 may be produced by amending the generated clinical note 10. For example, the generated clinical note 10 may be amended by the medical professional using the input device 150 to produce the amended clinical note 310. The amended clinical note 310 may include clinical data 312 at least partially written by the medical professional and at least partially produced by the production note generation model. The clinical data 312 may be in the form of text (e.g., phrases, sentences, paragraphs, and so forth).

The instructions 65 may further cause the processor 110 to extract, via the machine learning model 62, for each evaluation metric, a plurality of amended units 315 from the amended clinical note 310. In other words, for each evaluation metric, the machine learning model 62 may decompose the clinical data 312 of the amended clinical note 310 into the plurality of amended units 315. Each amended unit 316 from the plurality of amended units 315 may correspond to a respective evaluation metric from the at least one evaluation metric. In some embodiments, for one evaluation metric, each amended unit 316 may be a clinical concept. In some embodiments, for one evaluation metric, each amended unit 316 may be a SCU.

Referring to FIGS. 2, 3A, and 11, the instructions 65 may further cause the processor 110 to compare, via the machine learning model 62, for each evaluation metric, the plurality of generated units 15 and the plurality of amended units 315 corresponding to the evaluation metric. The instructions 65 may further cause the processor 110 to calculate, for each evaluation metric, an amended reference metric score 340 based on the comparison of the plurality of generated units 15 and the plurality of amended units 315 corresponding to the evaluation metric. The amended reference metric score 340 may be represented in terms of a fraction. The amended reference metric score 340 may range from 0 to 1, where 0 is the lowest score and 1 is the highest score.

Referring to FIGS. 2, 4, 11, and 12, in case of a plurality of evaluation metrics, the processor 110 may calculate a plurality of amended reference metric scores 339 corresponding to the plurality of evaluation metrics. For example, the plurality of amended reference metric scores 339 may include a first amended reference metric score 340A corresponding to a first evaluation metric, a second amended reference metric score 340B corresponding to a second evaluation metric, a third amended reference metric score 340C corresponding to a third evaluation metric, and so forth.

The instructions 65 may further cause the processor 110 to aggregate, via the machine learning model 62, the amended reference metric score 340 corresponding to each of the at least one evaluation metric to determine an amended reference quality score 345 of the generated clinical note 10. For example, the processor 110 may aggregate the plurality of amended reference metric scores 339, or more specifically, the first, second, and third amended reference metric scores 340A, 340B, 340C to determine the amended reference quality score 345 of the generated clinical note 10.

The instructions 65 may further cause the processor 110 to update the machine learning model 62 based on the quality score 45 and the amended reference quality score 345. Specifically, the processor 110 may update the machine learning model 62 (e.g., re-train the machine learning model 62, calibrate or tweak the machine learning model 62, change aggregation weights of the plurality of evaluation metrics, and so forth) to reduce a difference between the quality score 45 and the amended reference quality score 345. One problem that may arise in this approach is metric bias (i.e., prediction of high metric scores for production clinical notes), as some portions of the amended clinical note 310 will be substantially similar to the generated clinical note 10.

To overcome this problem, in some embodiments, the at least one evaluation metric may include at least one of a content factuality metric and a content coverage metric. The amended reference metric score 340 corresponding to each of the at least one of the content factuality metric and the content coverage metric may have a higher weight than the amended reference metric score 340 of the rest of the at least one evaluation metric for determining the amended reference quality score 345 of the generated clinical note 10. The content coverage metric and content factuality metric may produce less erroneous (i.e., less biased) amended reference metric scores 340 than other evaluation metrics.

The instructions 65 may further cause the processor 110 to output the updated machine learning model 62 to at least one of the storage device 130 and the non-transitory storage 120. This may allow the updated (and improved) machine learning model 62 to be used to predict the quality scores 45 of subsequent generated clinical notes.

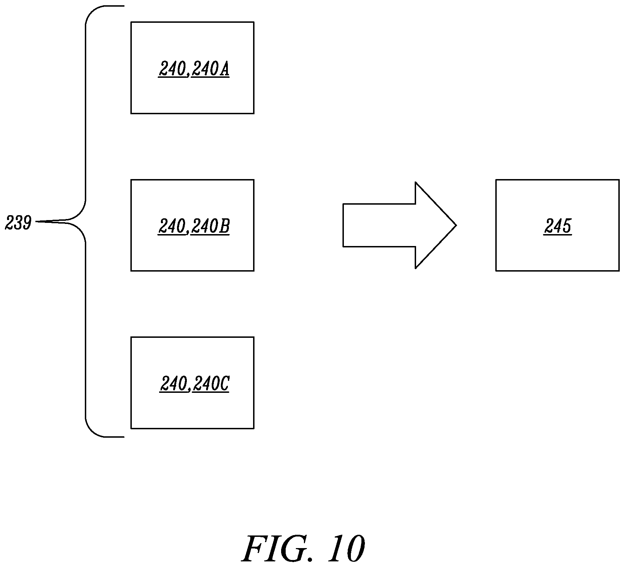

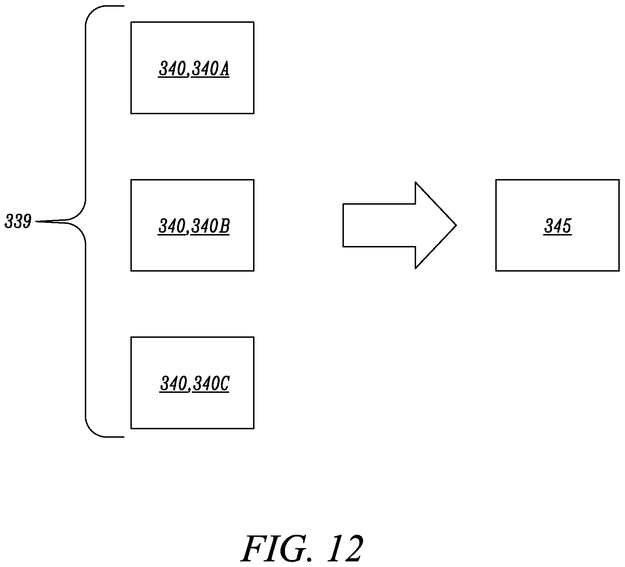

The machine learning model 62 may be continuously trained based on the draft reference metric scores 240 (shown in FIG. 10) and/or the amended reference metric scores 340 (shown in FIG. 12) to accurately determine the metric scores 40 (shown in FIG. 4), and continuously trained based on the draft reference quality score 245 and the amended reference quality score 345 to accurately predict the quality score 45 (shown in FIG. 4).

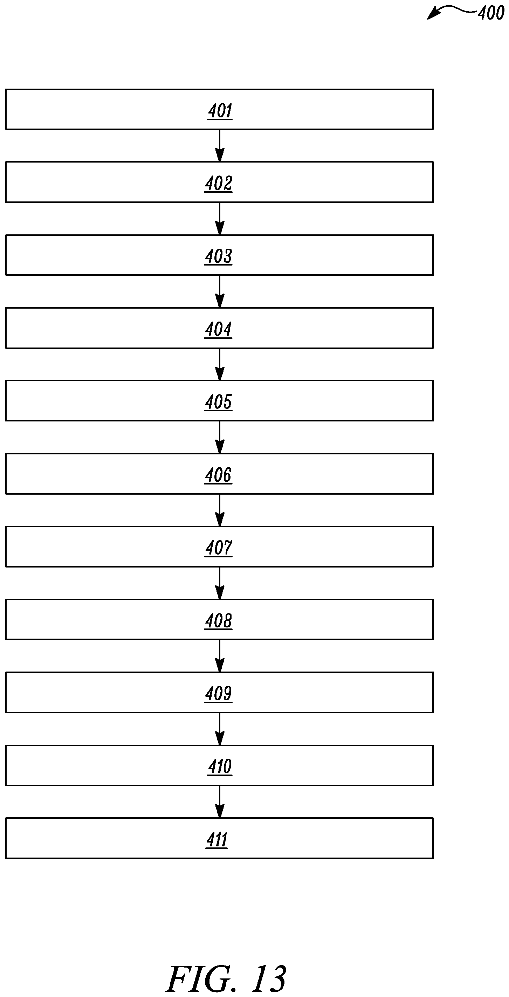

FIG. 13 illustrates a flowchart depicting various steps of a computer-implemented method 400 (hereinafter referred to as "the method 400") for evaluating generated clinical notes according to an embodiment of the present disclosure. The method 400 may be carried out by any suitable computing system, such as the system 100 of FIG. 1 and the system 101 of FIG. 2. The method 400 will be described with additional reference to FIGS. 2 to 12.

At step 401, the method 400 includes receiving a generated clinical note associated with a patient and a doctor-patient conversation (DoPaCo) transcript based on which the generated clinical note is generated. Referring to FIGS. 2 and 3A, for example, the method 400 may include receiving the generated clinical note 10 and the DoPaCo transcript 50.

At step 402, the method 400 further includes providing the DoPaCo transcript to a note generation model. Referring to FIG. 2, for example, the method 400 may include providing the DoPaCo transcript 50 to the note generation model 60.

At step 403, the method 400 further includes generating, via the note generation model, a plurality of pseudo-reference clinical notes based on the DoPaCo transcript. Referring to FIGS. 2 and 3B, for example, the method 400 may include generating, via the note generation model 60, the plurality of pseudo-reference clinical notes 20 based on the DoPaCo transcript 50.

At step 404, the method 400 further includes selecting at least one evaluation metric for evaluating the generated clinical note. The at least one evaluation metric may include metrics such as ROUGE-N (e.g., ROUGE-1, ROUGE-2, ROUGE-3, ROUGE-4) precision, ROUGE-N recall, ROUGE-N F1, ROUGE-L precision, ROUGE-L recall, ROUGE-L F1, clinical concept precision, clinical concept coverage/recall, Levenshtein distance, and Natural Language Inference (NLI) based metrics, such as content factuality and content coverage. In some examples, the generated clinical note may be evaluated using a plurality of the aforementioned metrics. Referring to FIG. 3A, for example, the method 400 may include selecting the at least one evaluation metric for evaluating the generated clinical note 10.

At step 405, the method 400 further includes extracting, via a machine learning model, for each evaluation metric from the at least one evaluation metric, a plurality of generated units from the generated clinical note. Referring to FIGS. 2 and 3A, for example, the method 400 may include extracting, via the machine learning model 62, for each evaluation metric from the at least one evaluation metric, the plurality of generated units 15 from the generated clinical note 10.

At step 406, the method 400 further includes extracting, via the machine learning model, for each evaluation metric, a plurality of sets of pseudo units from the plurality of pseudo-reference clinical notes. Each set of pseudo units from the plurality of sets of pseudo units is associated with a respective pseudo-reference clinical note from the plurality of pseudo-reference clinical notes. Each set of pseudo units includes a plurality of pseudo units.

Referring to FIGS. 2 and 3B, for example, the method 400 may include extracting, via the machine learning model 62, for each evaluation metric, the plurality of sets of pseudo units 30 from the plurality of pseudo-reference clinical notes 20. Each set of pseudo units 31 from the plurality of sets of pseudo units 30 is associated with a respective pseudo-reference clinical note 21 from the plurality of pseudo-reference clinical notes 20. Each set of pseudo units 31 includes the plurality of pseudo units 25.

At step 407, the method 400 further includes comparing, via the machine learning model, for each evaluation metric, the plurality of generated units and the plurality of pseudo units of each set of pseudo units corresponding to the evaluation metric. Referring to FIGS. 2, 3A, and 3B, for example, the method 400 may include comparing, via the machine learning model 62, the plurality of generated units 15 and the plurality of pseudo units 25 of each set of pseudo units 31 corresponding to the evaluation metric.

At step 408, the method 400 further includes calculating, for each evaluation metric, a plurality of sub-scores for the generated clinical note based on the comparison of the plurality of generated units and the plurality of pseudo units of each set of pseudo units corresponding to the evaluation metric. Each sub-score from the plurality of sub-scores corresponding to a respective evaluation metric is associated with a respective pseudo-reference clinical note.

Referring to FIGS. 2, 3A, 3B, and 4, for example, the method 400 may include calculating, for each evaluation metric, the plurality of sub-scores 35 for the generated clinical note 10 based on the comparison of the plurality of generated units 15 and the plurality of pseudo units 25 of each set of pseudo units 31 corresponding to the evaluation metric.

At step 409, the method 400 further includes aggregating, for each evaluation metric, the plurality of sub-scores corresponding to the evaluation metric to calculate a metric score corresponding to the evaluation metric. Referring to FIG. 4, for example, the method 400 may include aggregating, for each evaluation metric, the plurality of sub-scores 35 corresponding to the evaluation metric to calculate the metric score 40 corresponding to the evaluation metric.

At step 410, the method 400 further includes aggregating, via the machine learning model, the metric score corresponding to each of the at least one evaluation metric to predict a quality score of the generated clinical note. Referring to FIGS. 2 and 4, for example, the method 400 may include aggregating, via the machine learning model 62, the metric score 40 corresponding to each of the at least one evaluation metric to predict the quality score 45 of the generated clinical note 10. As another example, the method 400 may include aggregating, via the machine learning model 62, the first metric score 40A corresponding to the first evaluation metric and the second metric score 40B corresponding to the second evaluation metric to predict the quality score 45 of the generated clinical note 10.

At step 411, the method 400 further includes outputting, via an output device, the generated clinical note if the quality score is greater than or equal to a predetermined acceptable threshold, and a request to manually draft a drafted clinical note based on the DoPaCo transcript if the quality score is less than the predetermined acceptable threshold. Referring to FIGS. 2, 3A, 4, and 9, for example, the method 400 may include outputting, via the output device 140, the generated clinical note 10 if the quality score 45 is greater than or equal to the predetermined acceptable threshold, and the request to manually draft the drafted clinical note 210 based on the DoPaCo transcript 50 if the quality score 45 is less than the predetermined acceptable threshold.

The method 400 may employ the machine learning model to predict the quality score of the generated clinical note, which may correlate with an effort required to improve/correct the generated clinical note to an acceptable quality level. Specifically, the note generation model generates the plurality of pseudo-reference clinical notes, which is utilized by the machine learning model as missing human-written notes (since there are no human-written notes available in a production environment) corresponding to the DoPaCo transcript.

Further, the method 400 may evaluate the generated clinical note based on a plurality of evaluation metrics. Each evaluation metric may be related to an aspect of errors that contribute to extra effort/time for correcting the generated clinical note. Therefore, evaluating the generated clinical note based on the plurality of evaluation metrics may encompass various aspects that contribute to extra effort/time for correcting the generated clinical note and reduce metric bias (i.e., prediction of high metric scores for production clinical notes) of each individual evaluation metric.

In some embodiments, the at least one evaluation metric includes a clinical concept precision metric. Corresponding to the clinical concept precision metric, the plurality of generated units includes a plurality of generated clinical concepts and the plurality of pseudo units of each set of pseudo units includes a plurality of pseudo clinical concepts. For the clinical concept precision metric, each sub-score associated with the respective pseudo-reference clinical note is calculated based on a ratio of (i) a number of the plurality of generated clinical concepts that match with the plurality of pseudo clinical concepts of the set of pseudo units corresponding to the respective pseudo-reference clinical note to (ii) a total number of the plurality of generated clinical concepts of the generated clinical note.

In some embodiments, the at least one evaluation metric may include a clinical concept coverage metric. Corresponding to the clinical concept coverage metric, the plurality of generated units may include a plurality of generated clinical concepts and the plurality of pseudo units of each set of pseudo units may include a plurality of pseudo clinical concepts. For the clinical concept coverage metric, each sub-score associated with the respective pseudo-reference clinical note may be calculated based on a ratio of (i) a number of the plurality of pseudo clinical concepts of the set of pseudo units corresponding to the respective pseudo-reference clinical note that match with the plurality of generated clinical concepts of the generated clinical note to (ii) a total number of the plurality of pseudo clinical concepts of the set of pseudo units corresponding to the respective pseudo-reference clinical note.

In some embodiments, the at least one evaluation metric may include a content factuality metric. Corresponding to the content factuality metric, the plurality of generated units may include a plurality of generated SCUs and the plurality of pseudo units of each set of pseudo units may include a plurality of pseudo SCUs. For the content factuality metric, each sub-score associated with the respective pseudo-reference clinical note may be calculated by aggregating a plurality of generated entailment probabilities corresponding to the plurality of generated SCUs. Each generated entailment probability from the plurality of generated entailment probabilities may be associated with a respective generated SCU and corresponds to a maximum entailment probability of the respective generated SCU with respect to the plurality of pseudo SCUs of the respective pseudo-reference clinical note. Alternatively, for the content factuality metric, the top-k pseudo approach may be used to determine the sub-score associated with the respective pseudo-reference clinical note.

In some embodiments, the at least one evaluation metric may include a content coverage metric. Corresponding to the content coverage metric, the plurality of generated units may include a plurality of generated SCUs and the plurality of pseudo units of each set of pseudo units may include a plurality of pseudo SCUs. For the content coverage metric, each sub-score associated with the respective pseudo-reference clinical note may be calculated by aggregating a plurality of pseudo entailment probabilities corresponding to the plurality of pseudo SCUs of the respective pseudo-reference clinical note. Each pseudo entailment probability from the plurality of pseudo entailment probabilities may be associated with a respective pseudo SCU and corresponds to a maximum entailment probability of the respective pseudo SCU with respect to the plurality of generated SCUs of the generated clinical note. Alternatively, for the content coverage metric, the top-k generated approach may be used to determine the sub-score associated with the respective pseudo-reference clinical note.

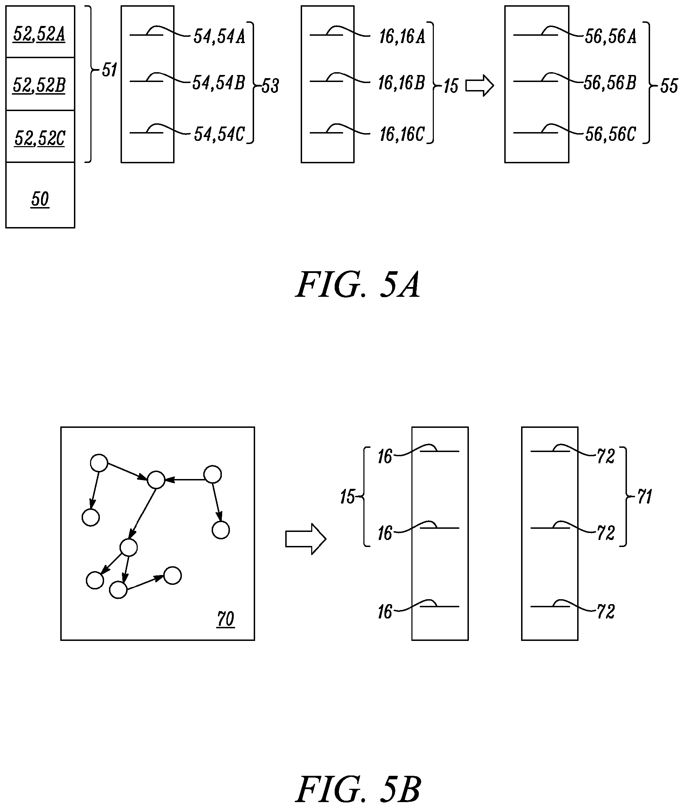

In some embodiments, the method 400 may further include receiving a plurality of transcript confidence scores corresponding to a plurality of transcript units of the DoPaCo transcript. Each transcript confidence score from the plurality of transcript confidence scores corresponds to a respective transcript unit from the plurality of transcript units. Referring to FIG. 5A, for example, the method 400 may include receiving the plurality of transcript confidence scores 53 corresponding to the plurality of transcript units 51 of the DoPaCo transcript 50.

The method 400 may further include determining, via the machine learning model, for one or more evaluation metrics from the at least one evaluation metric, which of the plurality of generated units correspond to which of the plurality of transcript units. Referring to FIGS. 2 and 5A, for example, the method 400 may include determining, via the machine learning model 62, for the one or more evaluation metrics, which of the plurality of generated units 15 correspond to which of the plurality of transcript units 51.

The method 400 may further include assigning, via the machine learning model, for the one or more evaluation metrics, a plurality of transcript weights to the plurality of generated units based on the plurality of transcript confidence scores. Referring to FIGS. 2 and 5A, for example, the method 400 may further include assigning, via the machine learning model 62, for the one or more evaluation metrics, the plurality of transcript weights 55 to the plurality of generated units 15 based on the plurality of transcript confidence scores 53.

For the one or more evaluation metrics, the plurality of sub-scores may be calculated further based on the plurality of transcript weights. Referring to FIGS. 2, 4, and 5A, in some embodiments, for the one or more evaluation metrics, the plurality of sub-scores 35 may be calculated further based on the plurality of transcript weights 55.

In some embodiments, the method 400 may further include assigning, for one or more evaluation metrics from the at least one evaluation metric, a plurality of knowledge weights to the plurality of generated units based on a knowledge graph of clinical data associated with the patient. Referring to FIG. 5B, for example, the method 400 may further include assigning, for the one or more evaluation metrics, the plurality of knowledge weights 71 to the plurality of generated units 15 based on the knowledge graph 70.

For the one or more evaluation metrics, the plurality of sub-scores may be calculated further based on the plurality of knowledge weights. Referring to FIGS. 4 and 5B, for example, for the one or more evaluation metrics, the plurality of sub-scores 35 may be calculated further based on the plurality of knowledge weights 71.

The method 400 may also provide explanations for the predicted quality score and hints for how the generated clinical note can be improved, for example, by highlighting portions of the generated clinical note that are predicted to be of low quality and adding missing data to the generated clinical note.

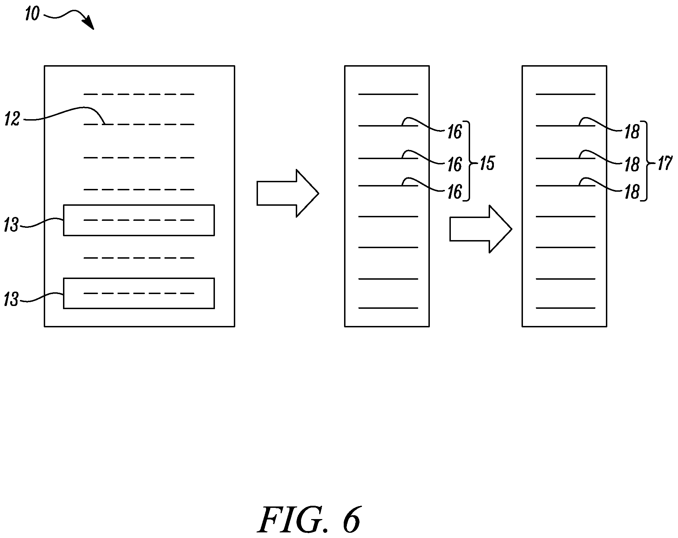

Specifically, in some embodiments, the method 400 may further include calculating, for one evaluation metric from the at least one evaluation metric, a plurality of unit scores for the plurality of generated units of the generated clinical note based on the comparison of the plurality of generated units and the plurality of pseudo units of each set of pseudo units corresponding to the one evaluation metric. Each unit score from the plurality of unit scores corresponds to a respective generated unit from the plurality of generated units. Referring to FIG. 6, for example, the method 400 may include calculating, for the one evaluation metric, the plurality of unit scores 17 for the plurality of generated units 15 of the generated clinical note 10.

The method 400 may further include determining which of the plurality of generated units have the unit scores less than a predetermined unit threshold. Referring to FIG. 6, for example, the method 400 may include determining which of the plurality of generated units 15 have the unit scores 18 less than the predetermined unit threshold.

The method 400 may further include determining one or more portions of the generated clinical note corresponding to the plurality of generated units having the unit scores less than the predetermined unit threshold. Referring to FIG. 6, for example, the method 400 may further include determining the one or more portions 13 of the generated clinical note 10 corresponding to the plurality of generated units 15 having the unit scores 18 less than the predetermined unit threshold.

The method 400 may further include highlighting the one or more portions of the generated clinical note corresponding to the plurality of generated units having the unit scores less than the predetermined unit threshold prior to outputting the generated clinical note. Referring to FIG. 6, for example, the method 400 may further include highlighting the one or more portions 13 of the generated clinical note 10 prior to outputting the generated clinical note 10.

Advantageously, the highlighted portions (i.e., the one or more portions) of the generated clinical note may help the medical professional in quickly identifying potential errors in the generated clinical note.

In some embodiments, the method 400 may further include comparing, via the machine learning model, for each evaluation metric, the plurality of pseudo units of the plurality of sets of pseudo units with each other. Referring to FIGS. 2 and 3B, for example, the method 400 may include comparing, via the machine learning model 62, for each evaluation metric, the plurality of pseudo units 25 of the plurality of sets of pseudo units 30 with each other.

The method 400 may further include determining, for each evaluation metric, a plurality of missing pseudo units from the plurality of pseudo units based on the comparison of the plurality of pseudo units of the plurality of sets of pseudo units with each other. The plurality of missing pseudo units may include the pseudo units that are missing equivalent generated units from the plurality of generated units and that repeat among the plurality of sets of pseudo units. Referring to FIG. 3B, for example, the method 400 may further include determining, for each evaluation metric, the plurality of missing pseudo units 27 from the plurality of pseudo units 25.

The method 400 may further include adding the plurality of missing pseudo units to the generated clinical note before outputting the generated clinical note if the quality score is greater than or equal to the predetermined acceptable threshold. Referring to FIGS. 3A and 3B, for example, the method 400 may further include adding the plurality of missing pseudo units 27 to the generated clinical note 10.

As a result, the generated clinical note may include missing clinical data (i.e., the plurality of missing pseudo units) when outputted via the output device. Advantageously, this may improve the quality of the generated clinical note when displayed to the medical professional. In some cases, the plurality of missing pseudo units added to the generated clinical note may be highlighted (or formatted differently from original clinical data of the generated note) to indicate that the plurality of missing pseudo units has been added to the generated note. The medical professional may therefore quickly identify and review the plurality of missing pseudo units added to the generated clinical note.

Moreover, the method 400 may employ quality prediction in a section-by-section manner. That is, the plurality of generated units of one section of the generated clinical note may be compared with the plurality of pseudo units of each of the plurality of pseudo-reference clinical notes of the same section, rather than comparing the generated clinical note and the plurality of pseudo-reference clinical notes in their entirety.

Specifically, in some embodiments, the generated clinical note may include a plurality of generated note sections and each of the plurality of pseudo-reference clinical notes may include a plurality of pseudo note sections corresponding to the plurality of generated note sections. The method 400 may further include comparing, via the machine learning model, for each evaluation metric, the plurality of generated units of each generated note section and the plurality of pseudo units of a respective pseudo note section corresponding to the generated note section of each set of pseudo units. Referring to FIGS. 2, 3A, 3B, 7A, and 7B, for example, the method 400 may include comparing, via the machine learning model 62, for each evaluation metric, the plurality of generated units 15 of each generated note section 81 and the plurality of pseudo units 25 of a respective pseudo note section 83 corresponding to the generated note section 81 of each set of pseudo units 31.

The method 400 may further include calculating, for each evaluation metric, a plurality of section sub-scores for each generated note section of the generated clinical note based on the comparison of the plurality of generated units of each generated note section and the plurality of pseudo units of the respective pseudo note section of each set of pseudo units. Each section sub-score from the plurality of section sub-scores corresponding to a respective evaluation metric may be associated with a respective pseudo-reference clinical note. Referring to FIGS. 2, 3A, 3B, 7A 7B, and 8, for example, the method 400 may include calculating, for each evaluation metric, the plurality of section sub-scores 84 for each generated note section 81 of the generated clinical note 10 based on the comparison of the plurality of generated units 15 of each generated note section 81 and the plurality of pseudo units 25 of the respective pseudo note section 83 of each set of pseudo units 31.

The method 400 may further include aggregating, for each evaluation metric, the plurality of section sub-scores for each generated note section to calculate a plurality of section scores for the plurality of generated note sections. Each generated note section has a corresponding section score from the plurality of section scores. Referring to FIGS. 7A and 8, for example, the method 400 may include aggregating, for each evaluation metric, the plurality of section sub-scores 84 for each generated note section 81 to calculate the plurality of section scores 86 for the plurality of generated note sections 80.

For each evaluation metric, the plurality of section scores may be aggregated with the plurality of sub-scores to calculate the metric score. Referring to FIGS. 4 and 8, for example, for each evaluation metric, the plurality of section scores 86 may be aggregated with the plurality of sub-scores 35 to calculate the metric score 40.

In some embodiments, the method 400 may further include modifying at least one generated note section from the plurality of generated note sections of the generated clinical note based on the section score of the at least one generated note section prior to outputting the generated clinical note. Modifying the at least one generated note section may include removing the at least one generated note section from the generated clinical note if the section score is less than a predetermined acceptable section threshold, and highlighting the at least one generated note section of the generated clinical note if the section score is greater than or equal to the predetermined acceptable section threshold. Referring to FIGS. 7A, 7B, and 8, for example, the method 400 may include modifying the at least one generated note section 81 from the plurality of generated note sections 80 of the generated clinical note 10 based on the section score 87 of the at least one generated note section 81 prior to outputting the generated clinical note 10. Modifying the at least one generated note section 81 may include removing the at least one generated note section 81 from the generated clinical note 10 if the section score 87 is less than the predetermined acceptable section threshold, and highlighting the at least one generated note section 81 of the generated clinical note 10 if the section score is greater than or equal to the predetermined acceptable section threshold.

Advantageously, if one generated note section has a very low quality (less than the predetermined acceptable section threshold) but rest of the plurality of generated note sections have acceptable quality (greater than the predetermined acceptable section threshold), the one generated note section may be removed from the generated clinical note, so that the one generated note section may be manually written by the medical professional. Further, if one generated note section has an acceptable quality (greater than the predetermined acceptable section threshold), but not a high quality, then the one generated note section may be highlighted for review.

As discussed above, the method 400 includes outputting, via the output device, the request to manually draft the drafted clinical note based on the DoPaCo transcript if the quality score is less than the predetermined acceptable threshold.

In some embodiments, the method 400 may further include receiving the drafted clinical note. The method 400 may further include extracting, via the machine learning model, for each evaluation metric, a plurality of drafted units from the drafted clinical note. Referring to FIGS. 2 and 9, for example, the method 400 may include receiving the drafted clinical note 210 and extracting, via the machine learning model 62, for each evaluation metric, the plurality of drafted units 215 from the drafted clinical note 210.

The method 400 may further include comparing, via the machine learning model, for each evaluation metric, the plurality of generated units and the plurality of drafted units corresponding to the evaluation metric. Referring to FIGS. 2, 3A, and 9, for example, the method 400 may include comparing, via the machine learning model 62, for each evaluation metric, the plurality of generated units 15 and the plurality of drafted units 215 corresponding to the evaluation metric.

The method 400 may further include calculating, for each evaluation metric, a draft reference metric score based on the comparison of the plurality of generated units and the plurality of drafted units corresponding to the evaluation metric. Referring to FIGS. 3A and 9, for example, the method 400 may include calculating, for each evaluation metric, the draft reference metric score 240 based on the comparison of the plurality of generated units 15 and the plurality of drafted units 215 corresponding to the evaluation metric.

The method 400 may further include aggregating, via the machine learning model, the draft reference metric score corresponding to each of the at least one evaluation metric to determine a draft reference quality score of the generated clinical note. Referring to FIGS. 2, 9, and 10, for example, the method 400 may include aggregating, via the machine learning model 62, the draft reference metric score 240 corresponding to each of the at least one evaluation metric to determine the draft reference quality score 245 of the generated clinical note 10.

The method 400 may further include updating the machine learning model based on the quality score and the draft reference quality score. The method 400 may further include outputting the updated machine learning model to at least one of a storage device and a non-transitory computer-readable storage. Referring to FIGS. 2, 4, and 10, for example, the method 400 may include updating the machine learning model 62 based on the quality score 45 and the draft reference quality score 245, and outputting the updated machine learning model 62 to at least one of the storage device 130 and the non-transitory storage 120.

Specifically, the method 400 may update the machine learning model (e.g., re-train the machine learning model, calibrate or tweak the machine learning model, change aggregation weights of the plurality of evaluation metrics, and so forth) to reduce a difference between the quality score and the draft reference quality score. One advantage of using the drafted clinical note for updating the machine learning model is that since the drafted clinical note is written from scratch by the medical practitioner, the draft reference quality score may be free from metric bias (i.e., prediction of high metric scores for production clinical notes), which may otherwise arise if a clinical note is produced by amending the generated clinical note. The method 400 may allow the updated (and improved) machine learning model to be used to predict the quality scores of subsequent generated clinical notes.

As discussed above, the method 400 includes outputting, via the output device, the generated clinical note (which may be modified before being output according to the present disclosure) if the quality score is greater than or equal to the predetermined acceptable threshold.

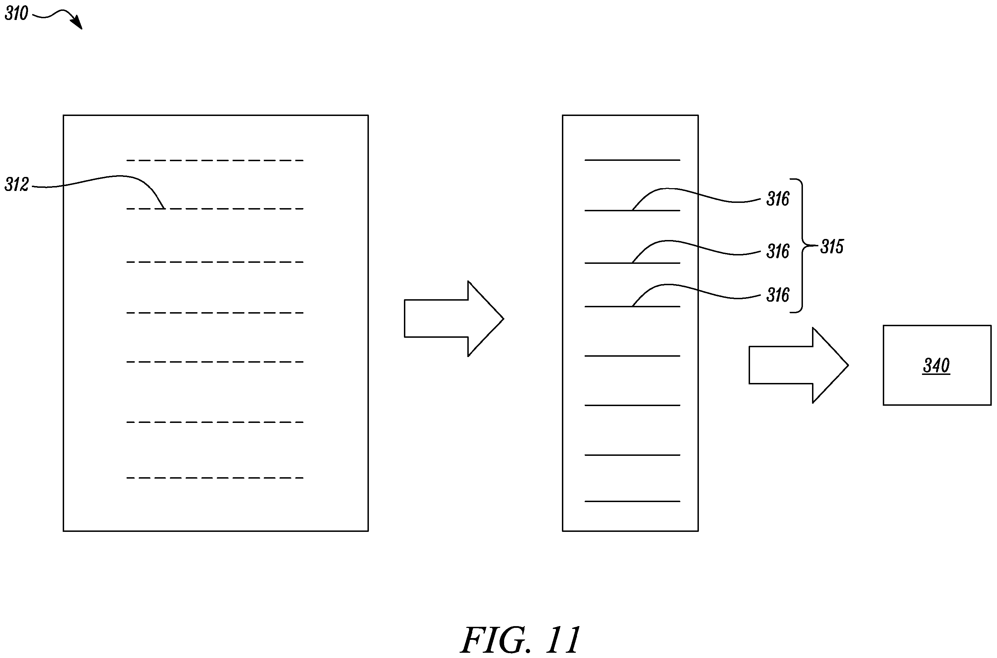

The method 400 may further include receiving an amended clinical note. The amended clinical note is produced by amending the generated clinical note. The method 400 may further include extracting, via the machine learning model, for each evaluation metric, a plurality of amended units from the amended clinical note. Referring to FIGS. 2 and 11, for example, the method 400 may include receiving the drafted clinical note 310 and extracting, via the machine learning model 62, for each evaluation metric, the plurality of amended units 315 from the amended clinical note 310.

The method 400 may further include comparing, via the machine learning model, for each evaluation metric, the plurality of generated units and the plurality of amended units corresponding to the evaluation metric. Referring to FIGS. 2, 3A, and 11, for example, the method 400 may include comparing, via the machine learning model 62, for each evaluation metric, the plurality of generated units 15 and the plurality of amended units 315 corresponding to the evaluation metric.

The method 400 may further include calculating, for each evaluation metric, an amended reference metric score based on the comparison of the plurality of generated units and the plurality of amended units corresponding to the evaluation metric. Referring to FIGS. 3A and 11, for example, the method 400 may include calculating, for each evaluation metric, the amended reference metric score 340 based on the comparison of the plurality of generated units 15 and the plurality of amended units 315 corresponding to the evaluation metric.

The method 400 may further include aggregating, via the machine learning model, the amended reference metric score corresponding to each of the at least one evaluation metric to determine an amended reference quality score of the generated clinical note. Referring to FIGS. 2, 11, and 12, for example, the method 400 may include aggregating, via the machine learning model 62, the amended reference metric score 340 corresponding to each of the at least one evaluation metric to determine the amended reference quality score 345 of the generated clinical note 10.

The method 400 may further include updating the machine learning model based on the quality score and the amended reference quality score. The method 400 may further include outputting the updated machine learning model to at least one of the storage device and the non-transitory computer-readable storage. Referring to FIGS. 2, 4, and 12, for example, the method 400 may include updating the machine learning model 62 based on the quality score 45 and the amended reference quality score 345, and outputting the updated machine learning model 62 to at least one of the storage device 130 and the non-transitory storage 120.

Specifically, the method 400 may update the machine learning model (e.g., re-train the machine learning model, calibrate or tweak the machine learning model, change aggregation weights of the plurality of evaluation metrics, and so forth) to reduce a difference between the quality score and the amended reference quality score. One problem that may arise in this approach is the metric bias (i.e., prediction of high metric scores for production clinical notes), as some portion of the amended clinical note will be substantially similar to the generated clinical note.

To overcome this problem, in some embodiments, the at least one evaluation metric may include at least one of a content factuality metric and a content coverage metric. The amended reference metric score corresponding to each of the at least one of the content factuality metric and the content coverage metric may have a higher weight than the amended reference metric score of the rest of the at least one evaluation metric for determining the amended reference quality score of the generated clinical note. The content coverage metric and content factuality metric may produce less erroneous (i.e., less biased) amended reference metric score than other evaluation metrics. Further, the method 400 may allow the updated (and improved) machine learning model 62 to be used to predict the quality scores of subsequent generated clinical notes.

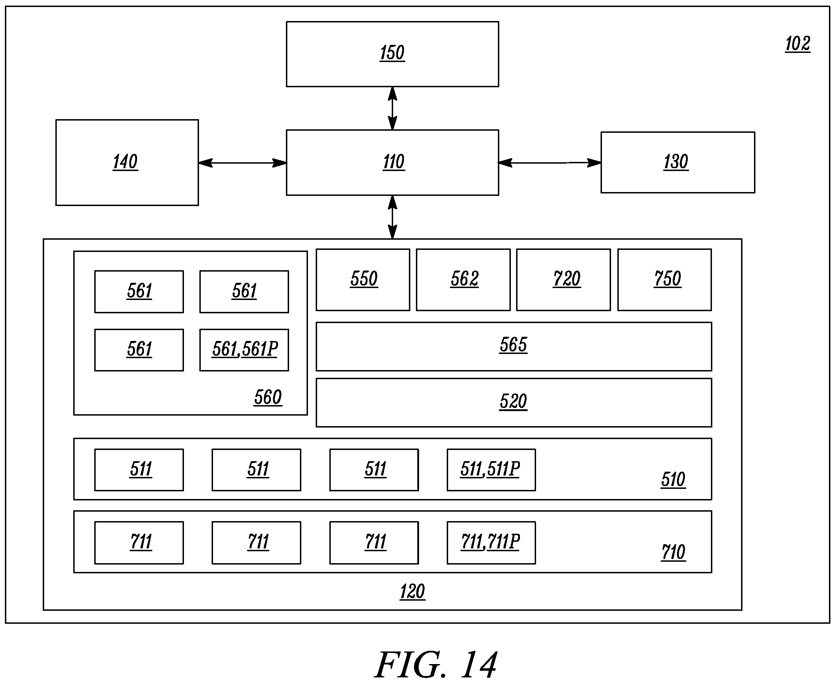

FIG. 14 illustrates a schematic block diagram of a system 102 for evaluating note generation models that generate clinical notes according to an embodiment of the present disclosure. The system 102 is similar to the system 100 of FIG. 1, with like elements designated by like reference characters.

In the illustrated embodiment of FIG. 14, the non-transitory storage 120 has stored thereon a plurality of note generation models 560, a machine learning model 562, and instructions 565. Each note generation model 561 from the plurality of note generation models 560 may be trained on a collection of historical DoPaCo transcripts, each of which is paired with a corresponding historical clinical note. That is, each note generation model 561 may be trained to produce generated clinical notes based on a DoPaCo transcript 550. The machine learning model 562 may be similar to the machine learning model 62 of FIG. 2 described above, i.e., the machine learning model 562 may be trained in a similar manner to the machine learning model 62 and possess similar functionalities to that of the machine learning model 62.

The instructions 565, when executed by the processor 110, cause the processor 110 to receive a plurality of generated clinical notes 510 generated by the corresponding plurality of note generation models 560 based on the DoPaCo transcript 550. Each generated clinical note 511 from the plurality of generated clinical notes 510 may be "AI generated," i.e., generated by a respective note generation model 561 from the plurality of note generation models 560. The plurality of note generation models 560 includes a production note generation model 561P that is currently used to generate clinical notes. The plurality of generated clinical notes 510 includes a production clinical note 511P that is generated by the production note generation model 561P.

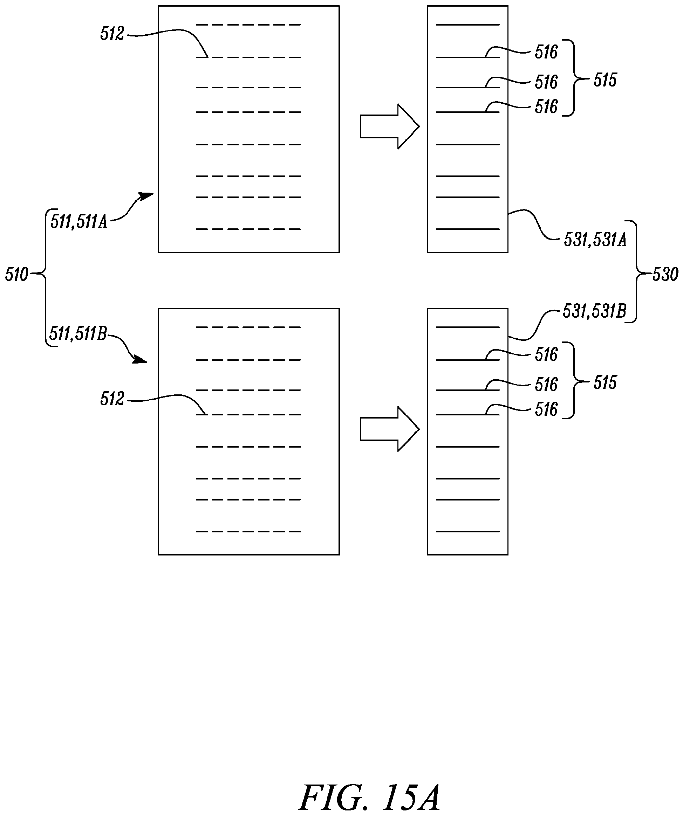

FIG. 15A schematically illustrates two generated clinical notes 511 from the plurality of generated clinical notes 510. Referring to FIGS. 14 and 15A, each generated clinical note 511 may include clinical data 512 derived from the DoPaCo transcript 550 by the respective note generation model 561. The clinical data 512 may be in the form of text (e.g., phrases, sentences, paragraphs, and so forth).

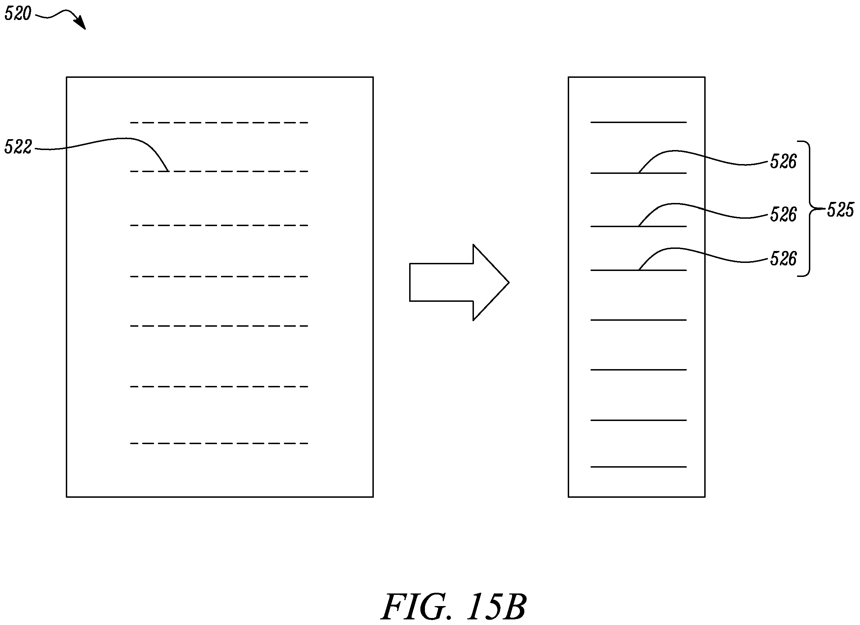

Referring to FIGS. 14 and 15B, the instructions 565 further cause the processor 110 to receive at least one final clinical note 520 that is approved by a medical professional based on the DoPaCo transcript 550. In some examples, the at least one final clinical note 520 may be manually drafted from scratch by the medical professional. In some other examples, the at least one final clinical note 520 may be an AI generated clinical note edited/amended by the medical professional. The at least one final clinical note 520 may include clinical data 522 derived from the DoPaCo transcript 550. The clinical data 522 may be in the form of text (e.g., phrases, sentences, paragraphs, and so forth).

The instructions 565 further cause the processor 110 to provide the plurality of generated clinical notes 510 and the at least one final clinical note 520 to the machine learning model 562.

The instructions 565 further cause the processor 110 to select a plurality of evaluation metrics for evaluating the plurality of generated clinical notes 510. Each generated clinical note 511 is evaluated based on the plurality of evaluation metrics. The plurality of evaluation metrics may include metrics such as ROUGE-N (e.g., ROUGE-1, ROUGE-2, ROUGE-3, ROUGE-4) precision, ROUGE-N recall, ROUGE-N F1, ROUGE-L precision, ROUGE-L recall, ROUGE-L F1, clinical concept precision, clinical concept coverage/recall, Levenshtein distance, and Natural Language Inference (NLI) based metrics, such as content factuality and content coverage.

Referring to FIGS. 14 and 15A, the instructions 565 further cause the processor 110 to extract, via the machine learning model 562, for each evaluation metric, a plurality of sets of generated units 530 from the plurality of generated clinical notes 510. Each set of generated units 531 from the plurality of sets of generated units 530 is associated with a respective generated clinical note 511 from the plurality of generated clinical notes 510. For example, the plurality of sets of generated units 530 may include a first set of generated units 531A associated with a first generated clinical note 511A from the plurality of generated clinical notes 510 and a second set of generated units 531B associated with a second generated clinical note 511B from the plurality of generated clinical notes 510. Each set of generated units 531 includes a plurality of generated units 515. In other words, for each evaluation metric, the machine learning model 562 may decompose the clinical data 512 of the respective generated clinical note 511 into the plurality of generated units 515. The plurality of generated units 515 may include two or more generated units 516.

Referring to FIGS. 14 and 15B, the instructions 565 further cause the processor 110 to extract, via the machine learning model 562, for each evaluation metric, a plurality of final units 525 from the at least one final clinical note 520. In other words, for each evaluation metric, the machine learning model 562 may decompose the clinical data 522 of the at least one final clinical note 511 into the plurality of final units 525. The plurality of final units 525 may include two or more final units 526.

Referring to FIGS. 14, 15A, and 15B, the instructions 565 further cause the processor 110 to compare, via the machine learning model 562, for each evaluation metric, the plurality of generated units 515 of each set of generated units 531 and the plurality of final units 525 of the at least one final clinical note 520 corresponding to the evaluation metric.

Referring to FIGS. 14, 15A, 15B, and 16, the instructions 565 further cause the processor 110 to calculate, for each evaluation metric, a metric score 540 for each of the plurality of generated clinical notes 510 based on the comparison of the plurality of generated units 515 of each set of generated units 531 and the plurality of final units 525 of the at least one final clinical note 520 corresponding to the evaluation metric. The metric score 540 may be represented in terms of a fraction. The metric score 540 may range from 0 to 1, where 0 is the lowest score and 1 is the highest score.

For example, the processor 110 may calculate, for a first evaluation metric from the plurality of evaluation metrics, a first metric score 540A1 for the first generated clinical note 511A based on the comparison of the plurality of generated units 515 of the set of generated units 531 corresponding to the first generated clinical note 511A and the plurality of final units 525 of the at least one final clinical note 520 corresponding to the first evaluation metric. Further, the processor 110 may calculate, for a second evaluation metric from the plurality of evaluation metrics, a second metric score 540A2 for the first generated clinical note 511A based on the comparison of the plurality of generated units 515 of the set of generated units 531 corresponding to the first generated clinical note 511A and the plurality of final units 525 of the at least one final clinical note 520 corresponding to the second evaluation metric.

In a similar manner, the processor 110 may calculate, for the first evaluation metric, a first metric score 540B1 for the second generated clinical note 511B, and for the second evaluation metric, a second metric score 540B2 for the second generated clinical note 511B. Likewise, the processor 110 may calculate, for the first evaluation metric, a first metric score 540C1 for the production clinical note 511P, and for the second evaluation metric, a second metric score 540C2 for the production clinical note 511P.

The instructions 565 further cause the processor 110 to aggregate, via the machine learning model 562, for each generated clinical note 511, the metric scores 540 corresponding to the plurality of evaluation metrics to determine a model score 545 associated with the note generation model 561 that generated the generated clinical note 511.

For example, for the first generated clinical note 511A, the processor 110 may aggregate, via the machine learning model 562, the first metric score 540A1 corresponding to the first evaluation metric and the second metric score 540A2 corresponding to the second evaluation metric to determine a model score 545A associated with the note generation model 561 that generated the first generated clinical note 511A.

Similarly, for the second generated clinical note 511B, the processor 110 may aggregate, via the machine learning model 562, the first metric score 540B1 corresponding to the first evaluation metric and the second metric score 540B2 corresponding to the second evaluation metric to determine a model score 545B associated with the note generation model 561 that generated the second generated clinical note 511B.

Similarly, for the production clinical note 511P, the processor 110 may aggregate, via the machine learning model 562, the first metric score 540C1 corresponding to the first evaluation metric and the second metric score 540C2 corresponding to the second evaluation metric to determine a model score 545P associated with the production note generation model 561P that generated the production clinical note 511P.

The instructions 565 further cause the processor 110 to select one of the plurality of note generation models 560 to generate clinical notes at least partially based on the model scores 545.

In some embodiments, the instructions 565 may further cause the processor 110 to select the note generation model 561 having the highest model score 545 to generate clinical notes instead of the production note generation model 561P if the highest model score 545 is greater than the model score 545P of the production note generation model 561P by a predetermined margin. For example, if the predetermined margin is 10%, the processor 110 may select the note generation model 561 having the highest model score 545, if the highest model score 545 is greater than 110% of the model score 545P of the production note generation model 561P.

The system 102 may evaluate the plurality of note generation models 560 based on the plurality of evaluation metrics with respect to the at least one final clinical note 520. In some examples, the at least one final clinical note 520 may include a plurality of final clinical notes 520 approved by a corresponding plurality of medical professionals. This may allow aggregating the model scores 545 determined for the plurality of final clinical notes 520. Using the plurality of final clinical notes 520 for evaluation may allow a more comprehensive evaluation of the plurality of note generation models 560, as different medical professionals may produce clinical notes in various different manners (writing style, formatting, terminology used, etc.).

In some embodiments, the at least one final clinical note 520 may be produced by amending one generated clinical note 511 from the plurality of generated clinical notes 510. For example, the at least one final clinical note 520 may be produced by amending the production clinical note 511P.

In such embodiments, the plurality of evaluation metrics may include at least one of a content factuality metric and a content coverage metric. For each generated clinical note 511, the metric score 540 corresponding to each of the at least one of the content factuality metric and the content coverage metric may have a higher weight than the metric score 540 of the rest of the plurality of evaluation metrics for determining the model score 545 associated with the generated clinical note 511.

In examples where the at least one final clinical note 520 is produced by amending the production clinical note 511P, some metrics may exhibit a bias towards the production note generation model 561P, that is, the model score 545 for the production note generation model 561P may be high. The content factuality metric and the content coverage metric may be less biased than others of the plurality of evaluation metrics for evaluation in such cases, and therefore may be preferably assigned higher aggregation weights for determining the model score 545 for each note generation model 561. Thus, in such cases, it may be beneficial to rely more heavily on evaluation metrics such as the content factuality metric and the content coverage metric to obtain accurate model scores 545.

Some medical professionals may prefer using the note generation models 561 that are determined to be more precise/conservative (i.e., having high model scores 545) to generate clinical notes, but others may prefer using the note generation models 561 that create as much content as possible. To that end, in some examples, weighting of the plurality of evaluation metrics may be user-selected. In some cases, users may manually set different weights for factual errors in different sections of clinical notes (e.g., Assessment and Plan weighted higher than History of Present Illness). As a result, the system 102 may select the note generation model 561 that is best suited for a medical professional based on their preference. In some examples, the at least one final clinical note 520 may be manually drafted by the medical professional from scratch. In such cases, the at least one final clinical note 520 may also be used to adjust the weightage of the plurality of evaluation metrics, i.e., to fine-tune/calibrate the machine learning model 562. In some embodiments, the machine learning model 562 may determine a severity of factual errors and assign these a higher weight. For example, the machine learning model 562 may employ a medical concept engine to detect references to important medical entities (e.g., medication) in SCUs with poor factuality scores, and raise the weightage on these units.

In some embodiments, corresponding to the content factuality metric, the plurality of generated units 515 of each set of generated units 531 may include a plurality of generated SCUs and the plurality of final units 525 of the at least one final clinical note 520 may include a plurality of final SCUs. For the content factuality metric, the metric score 540 for a respective generated clinical note 511 from the plurality of generated clinical notes 510 may be calculated by aggregating a plurality of generated entailment probabilities corresponding to the plurality of generated SCUs of the set of generated units 531 associated with the respective generated clinical note 511. Each generated entailment probability from the plurality of generated entailment probabilities may be associated with a respective generated SCU and correspond to a maximum entailment probability of the respective generated SCU with respect to the plurality of final SCUs of the at least one final clinical note 520.

In some embodiments, corresponding to the content coverage metric, the plurality of generated units 515 of each set of generated units 531 may include a plurality of generated SCUs and the plurality of final units 525 of the at least one final clinical note 520 may include a plurality of final SCUs. For the content coverage metric, the metric score 540 for a respective generated clinical note 511 from the plurality of generated clinical notes 510 may be calculated by aggregating a plurality of final entailment probabilities corresponding to the plurality of final SCUs of the at least one final clinical note 520. Each final entailment probability from the plurality of final entailment probabilities may be associated with a respective final SCU and correspond to a maximum entailment probability of the respective final SCU with respect to the plurality of generated SCUs of set of generated SCUs corresponding to the generated clinical note 511.

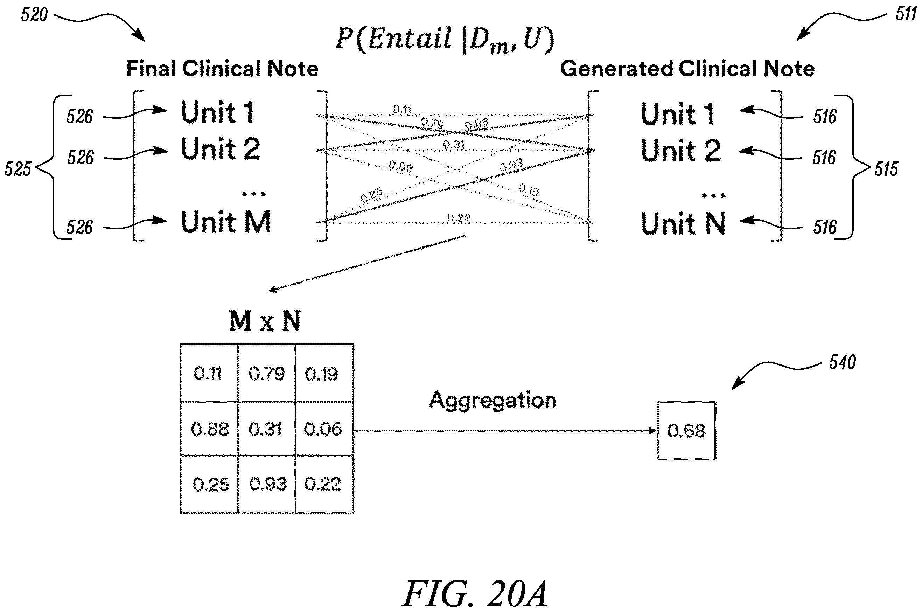

FIG. 20A schematically illustrates a flow diagram depicting steps for determining the metric score 540 for one generated clinical note 511 corresponding to the content factuality metric according to an embodiment of the present disclosure. Referring to FIG. 20A, the plurality of final units 525 includes M final units 526, and the plurality of generated units 515 includes N generated units 516. Each M final unit 526 corresponds to a final SCU and each N generated unit 516 corresponds to a generated SCU.

For the content factuality metric, the plurality of final units 525 may be considered as NLI premise, and each generated unit 516 may be considered as NLI hypothesis. The machine learning model 562 (shown in FIG. 14) may determine probabilities of entailment for all MxN pairs, thereby producing a MxN matrix. For the content factuality metric, the MxN matrix may be reduced to a single scalar value (i.e., the metric score 540). One approach may include selecting the maximum value over each column of the MxN matrix, corresponding to selecting the most probable evidence in favor of any generated unit 516, followed by taking a mean over the resulting values. Other variations of this are possible, up to and including, training an additional model to aggregate the scores.

For the content coverage metric, the plurality of generated units 515 may be considered as NLI premise, and each final unit 526 may be considered as NLI hypothesis. The machine learning model 562 (shown in FIG. 14) may determine probabilities of entailment for all NxM pairs, thereby producing a NxM matrix. For the content coverage metric, the NxM matrix may be reduced to a single scalar value (i.e., the metric score 540). One approach may include selecting the maximum value over each column of the NxM matrix, corresponding to selecting the most probable evidence in favor of any final unit 526, followed by taking a mean over the resulting values. Other variations of this are possible, up to and including training an additional model to aggregate the scores.

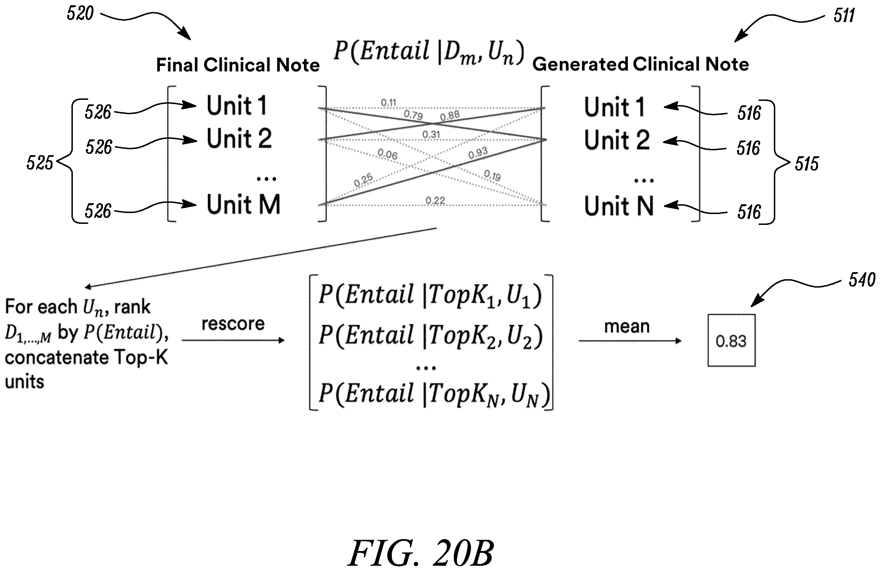

FIG. 20B schematically illustrates a flow diagram depicting steps for determining the metric score 540 for one generated clinical note 511 corresponding to a top-k factuality metric according to an embodiment of the present disclosure. In some embodiments, the content factuality metric may include the top-k factuality metric.

Referring to FIG. 20B, the plurality of final units 525 includes M final units 526, and the plurality of generated units 515 includes N generated units 516. Each M final unit 526 corresponds to a final SCU and each N generated unit 516 corresponds to a generated SCU. For each generated unit 516, top final units 526 may be determined, where the top final units 526 include the final units 526 having maximum entailment probabilities greater than a predetermined entailment threshold with respect to the generated unit 516. Further, for each generated unit 516, the top final units 526 may be concatenated to form a "top-k final premise." This may allow dynamically increasing the context or premise using only the most relevant information.

For the top-k factuality metric, for each generated unit 516, the entailment score of the generated unit 516 may be determined by considering the generated unit 516 as the "hypothesis" against the determined top-k final premise. Similarly, the entailment scores for the plurality of generated units 515 may be calculated and aggregated (e.g., averaged) to determine, for the top-k factuality metric, the metric score 540 for the one generated clinical note 511.

In some embodiments, a top-k coverage metric may be used to determine the metric score 540 for the one generated clinical note 511. In some embodiments, the content coverage metric may include the top-k coverage metric. Specifically, for each final unit 526, top generated units 516 may be determined, where the top generated units 516 include the generated units 516 having maximum entailment probabilities greater than a predetermined entailment threshold with respect to the final unit 526. For each final unit 526, the top generated units 516 may be concatenated to form a "top-k generated premise." For the top-k coverage metric, for each final unit 526, the entailment score of the final unit 526 may be determined by considering the final unit 526 as the "hypothesis" against the determined top-k generated premise. Similarly, the entailment scores for the plurality of final units 525 may be calculated and aggregated (e.g. averaged) to determine, for the top-k coverage metric, the metric score 540 for the one generated clinical note 511.

As discussed above, the content factuality metric and the content coverage metric may be more reliable than other evaluation metrics in some cases, and thus may be assigned higher weights when calculating the model scores 545 for the plurality of note generation models 560.

It may be noted that the plurality of note generation models 560 may also be evaluated using a plurality of DoPaCo transcripts (such as the DoPaCo transcript 550).

Specifically, referring to FIG. 14, the instructions 565 may further cause the processor 110 to receive a plurality of second generated clinical notes 710. The plurality of second generated clinical notes 710 may be generated by the corresponding plurality of note generation models 560 based on a second DoPaCo transcript 750. The plurality of second generated clinical notes 710 may include a second production clinical note 711P that is generated by the production note generation model 561P.

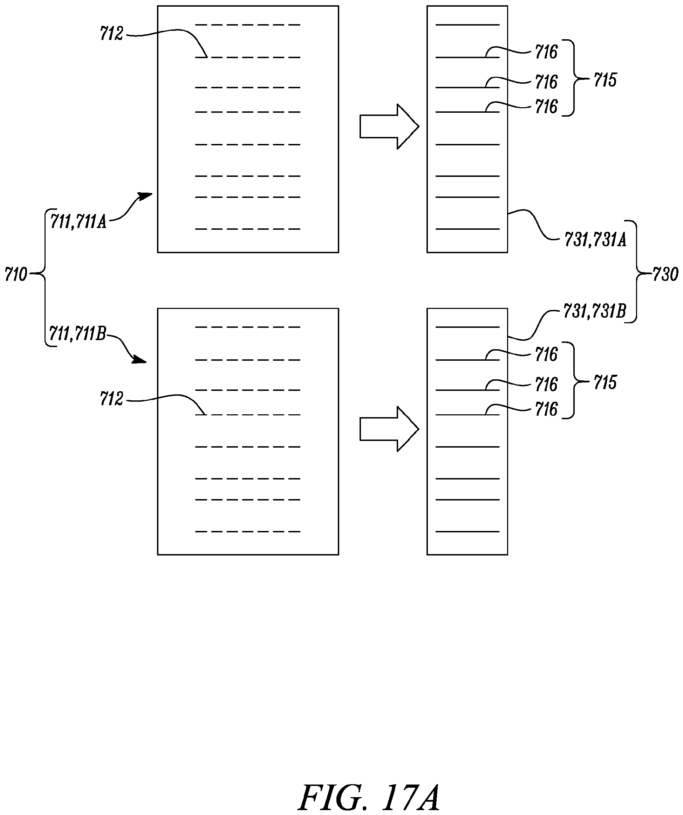

FIG. 17A schematically illustrates two second generated clinical notes 711 from the plurality of second generated clinical notes 710. Referring to FIGS. 14 and 17A, each second generated clinical note 711 may include clinical data 712 derived from the second DoPaCo transcript 750 by the respective note generation model 561. The clinical data 712 may be in the form of text (e.g., phrases, sentences, paragraphs, and so forth).

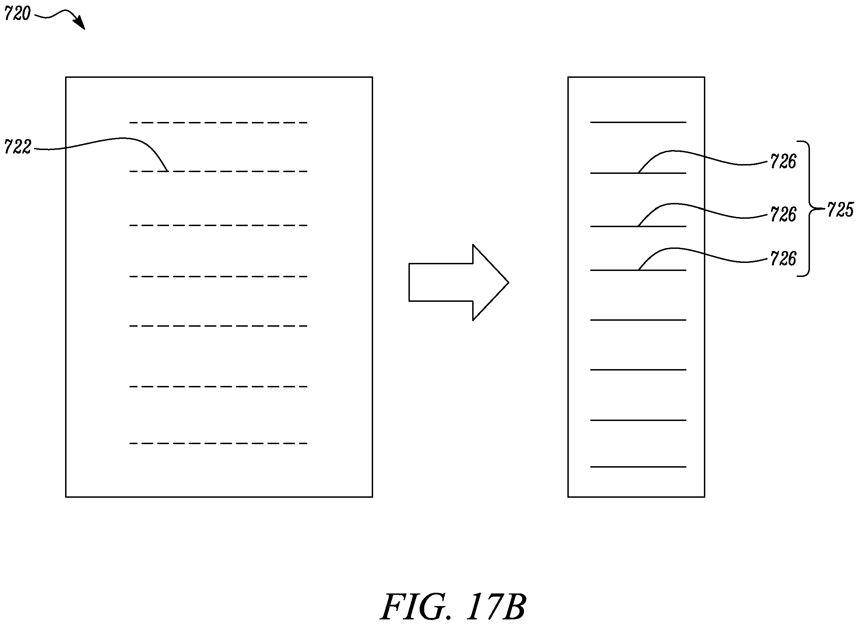

Referring to FIGS. 14 and 17B, the instructions 565 may further cause the processor 110 to receive at least one second final clinical note 720 that is approved by the medical professional based on the second DoPaCo transcript 750. In some examples, the at least one second final clinical note 720 may be manually drafted from scratch by the medical professional. In some other examples, the at least one second final clinical note 720 may be an AI generated clinical note edited/amended by the medical professional. The at least one second final clinical note 720 may include clinical data 722 derived from the second DoPaCo transcript 750. The clinical data 722 may be in the form of text (e.g., phrases, sentences, paragraphs, and so forth).

The instructions 565 may further cause the processor 110 to provide the plurality of second generated clinical notes 710 and the at least one second final clinical note 720 to the machine learning model 562.

Referring to FIGS. 14 and 17A, the instructions 565 may further cause the processor 110 to extract, via the machine learning model 562, for each evaluation metric, a plurality of sets of second generated units 730 from the plurality of second generated clinical notes 710. Each set of second generated units 731 from the plurality of sets of second generated units 730 may be associated with a respective second generated clinical note 711 from the plurality of second generated clinical notes 710. For example, the plurality of sets of second generated units 730 may include a first set of second generated units 731A associated with a second generated clinical note 711A from the plurality of second generated clinical notes 710 and a second set of second generated units 731B associated with a second generated clinical note 711B from the plurality of second generated clinical notes 710. Each set of second generated units 731 may include a plurality of second generated units 715. In other words, for each evaluation metric, the machine learning model 562 may decompose the clinical data 712 of the respective second generated clinical note 711 into the plurality of second generated units 715. The plurality of second generated units 715 may include two or more second generated units 716.

Referring to FIGS. 14 and 17B, the instructions 565 may further cause the processor 110 to extract, via the machine learning model 562, for each evaluation metric, a plurality of second final units 725 from the at least one second final clinical note 720. In other words, for each evaluation metric, the machine learning model 562 may decompose the clinical data 722 of the at least one second final clinical note 711 into the plurality of second final units 725. The plurality of second final units 725 may include two or more second final units 726.

Referring to FIGS. 14, 17A, and 17B, the instructions 565 may further cause the processor 110 to compare, via the machine learning model 562, for each evaluation metric, the plurality of second generated units 715 of each set of second generated units 731 and the plurality of second final units 725 of the at least one second final clinical note 720 corresponding to the evaluation metric.

Referring to FIGS. 14, 17A, 17B, and 18, the instructions 565 may further cause the processor 110 to calculate, for each evaluation metric, a second metric score 740 for each of the plurality of second generated clinical notes 711 based on the comparison of the plurality of second generated units 715 of each set of second generated units 731 and the plurality of second final units 725 of the at least one second final clinical note 720 corresponding to the evaluation metric. The second metric score 740 may be represented in terms of a fraction. The second metric score 740 may range from 0 to 1, where 0 is the lowest score and 1 is the highest score.

The second metric score 740 for each of plurality of second generated clinical notes 711 may be determined similarly to the metric score 540 described above. As an example, the processor 110 may calculate, for a first evaluation metric from the plurality of evaluation metrics, a second metric score 740A1 for the second generated clinical note 711A, and for a second evaluation metric from the plurality of evaluation metrics, a second metric score 740A2 for the second generated clinical note 711A. In a similar manner, the processor 110 may calculate, for the first evaluation metric, a second metric score 740B1 for the second generated clinical note 711B, and for a second evaluation metric, a second metric score 740B2 for the second generated clinical note 711B. Likewise, the processor 110 may calculate, for the first evaluation metric, a second metric score 740C1 for the second production clinical note 711P, and for the second evaluation metric, a second metric score 740C2 for the second production clinical note 711P.

The instructions 565 may further cause the processor 110 aggregate, via the machine learning model 562, for each second generated clinical note 711, the second metric scores 740 corresponding to the plurality of evaluation metrics to determine a second model score 745 associated with the note generation model 561 that generated the second generated clinical note 711.

For example, for the second generated clinical note 711A, the processor 110 may aggregate, via the machine learning model 562, the second metric score 740A1 corresponding to the first evaluation metric and the second metric score 740A2 corresponding to the second evaluation metric to determine a second model score 745A associated with the note generation model 561 that generated the second generated clinical note 711A. Similarly, the processor 110 may aggregate, via the machine learning model 562, the second metric score 740B1 corresponding to the first evaluation metric and the second metric score 740B2 corresponding to the second evaluation metric to determine a second model score 745B associated with the note generation model 561 that generated the second generated clinical note 711B. Likewise, the processor 110 may aggregate, via the machine learning model 562, the second metric score 740C1 corresponding to the first evaluation metric and the second metric score 740C2 corresponding to the second evaluation metric to determine a second model score 745P associated with the production note generation model 561P that generated the second production clinical note 711P.

Referring to FIGS. 14, 16, and 18, the instructions 565 may further cause the processor 110 aggregate, for each note generation model 561, the model score 545 and the second model score 745 to determine an aggregated model score 546 for the note generation model 561. For example, the processor 110 may aggregate, for the note generation model 561 that generated the first generated clinical note 511A and the second generated clinical note 711A, the model score 545A and the second model score 745A to determine an aggregated model score 546A for the note generation model 561. Similarly, the processor 110 may aggregate, for the note generation model 561 that generated the second generated clinical note 511B and the second generated clinical note 711B, the model score 545B and the second model score 745B to determine an aggregated model score 546B for the note generation model 561. Likewise, the processor 110 may aggregate, for the production note generation model 561P that generated the production generated clinical note 511P and the second production generated clinical note 711P, the model score 545P and the second model score 745P to determine an aggregated model score 546P for the production note generation model 561P.

In some embodiments, selecting one of the plurality of note generation models 560 to generate clinical notes is at least partially based on the aggregated model scores 546.

In some embodiments, the processor 110 may select the note generation model 561 having the highest aggregate model score 546 to generate clinical notes instead of the production note generation model 561P if the highest aggregated model score 546 is greater than the aggregated model score 546P of the production note generation model 561P by a predetermined margin. For example, if the predetermined margin is 10%, the processor 110 may select the note generation model 561 having the highest aggregated model score 546, if the highest aggregated model score 546 is greater than 110% of the aggregated model score 546P of the production note generation model 561P.

In a similar manner, the plurality of note generation models 560 may be evaluated using a plurality of DoPaCo transcripts (such as the DoPaCo transcript 550). Specifically, the plurality of DoPaCo transcripts may be used determine aggregated model scores 546 and select one of the plurality of note production models 560 to generate clinical notes.

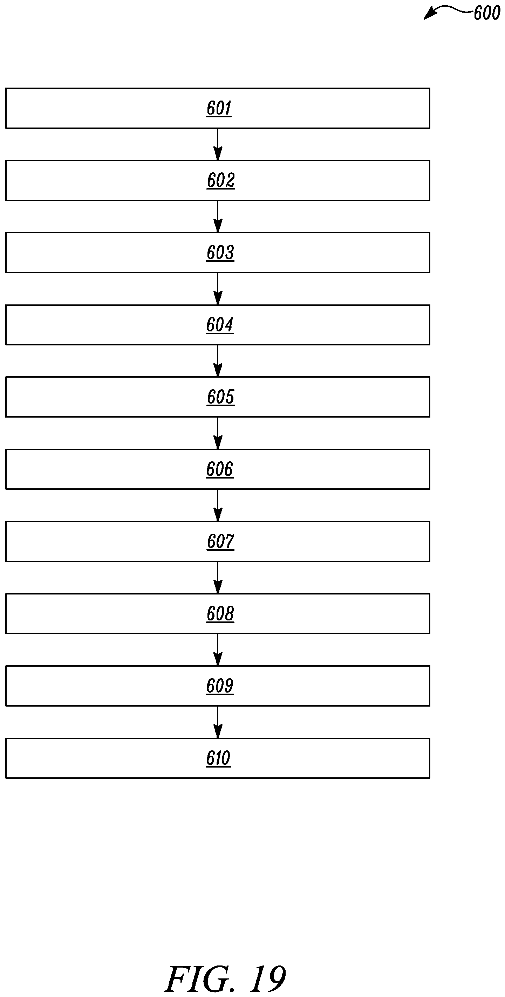

FIG. 19 illustrates a flowchart depicting various steps of a computer-implemented method 600 (hereinafter referred to as "the method 600") for evaluating note generation models that generate clinical notes according to an embodiment of the present disclosure. The method 600 may be carried out by any suitable computing system, such as the system 100 of FIG. 1 and the system 102 of FIG. 14. The method 600 will be described with additional reference to FIGS. 14 to 18.

At step 601, the method 600 includes receiving a plurality of generated clinical notes. The plurality of generated clinical notes is generated by a corresponding plurality of note generation models based on a DoPaCo transcript. The plurality of note generation models includes a production note generation model that is currently used to generate clinical notes. The plurality of generated clinical notes includes a production clinical note that is generated by the production note generation model. Referring to FIG. 14, for example, the method 600 may include receiving the plurality of generated clinical notes 510.

At step 602, the method 600 further includes receiving at least one final clinical note that is approved by the medical professional based on the DoPaCo transcript. Referring to FIG. 14, for example, the method 600 may include receiving the at least one final clinical note 520.

At step 603, the method 600 further includes providing the plurality of generated clinical notes and the at least one final clinical note to a machine learning model. Referring to FIG. 14, for example, the method 600 may include providing the plurality of generated clinical notes 510 and the at least one final clinical note 520 to the machine learning model 562.

At step 604, the method 600 further includes selecting a plurality of evaluation metrics for evaluating the plurality of generated clinical notes. The plurality of evaluation metrics may include metrics such as ROUGE-N (e.g., ROUGE-1, ROUGE-2, ROUGE-3, ROUGE-4) precision, ROUGE-N recall, ROUGE-N F1, ROUGE-L precision, ROUGE-L recall, ROUGE-L F1, clinical concept precision, clinical concept coverage/recall, Levenshtein distance, and Natural Language Inference (NLI) based metrics, such as content factuality and content coverage.

At step 605, the method 600 further includes extracting, via the machine learning model, for each evaluation metric from the plurality of evaluation metrics, a plurality of sets of generated units from the plurality of generated clinical notes. Each set of generated units from the plurality of sets of generated units is associated with a respective generated clinical note from the plurality of generated clinical notes. Each set of generated units includes a plurality of generated units. Referring to FIGS. 14 and 15A, for example, the method 600 may include extracting, via the machine learning model 562, for each evaluation metric, the plurality of sets of generated units 530 from the plurality of generated clinical notes 510.

At step 606, the method 600 further includes extracting, via the machine learning model, for each evaluation metric, a plurality of final units from the at least one final clinical note. Referring to FIGS. 14 and 15B, for example, the method 600 may include extracting, via the machine learning model 562, for each evaluation metric, the plurality of final units 525 from the at least one final clinical note 520.

At step 607, the method 600 further includes comparing, via the machine learning model, for each evaluation metric, the plurality of generated units of each set of generated units and the plurality of final units of the at least one final clinical note corresponding to the evaluation metric. Referring to FIGS. 14, 15A, and 15B, for example, the method 600 may include comparing, via the machine learning model 562, for each evaluation metric, the plurality of generated units 515 of each set of generated units 531 and the plurality of final units 525 of the at least one final clinical note 520 corresponding to the evaluation metric.

At step 608, the method 600 further includes calculating, for each evaluation metric, a metric score for each of the plurality of generated clinical notes based on the comparison of the plurality of generated units of each set of generated units and the plurality of final units of the at least one final clinical note corresponding to the evaluation metric. Referring to FIGS. 14, 15A, 15B, and 16, for example, the method 600 may include calculating, for each evaluation metric, the metric score 540 for each of the plurality of generated clinical notes 511.

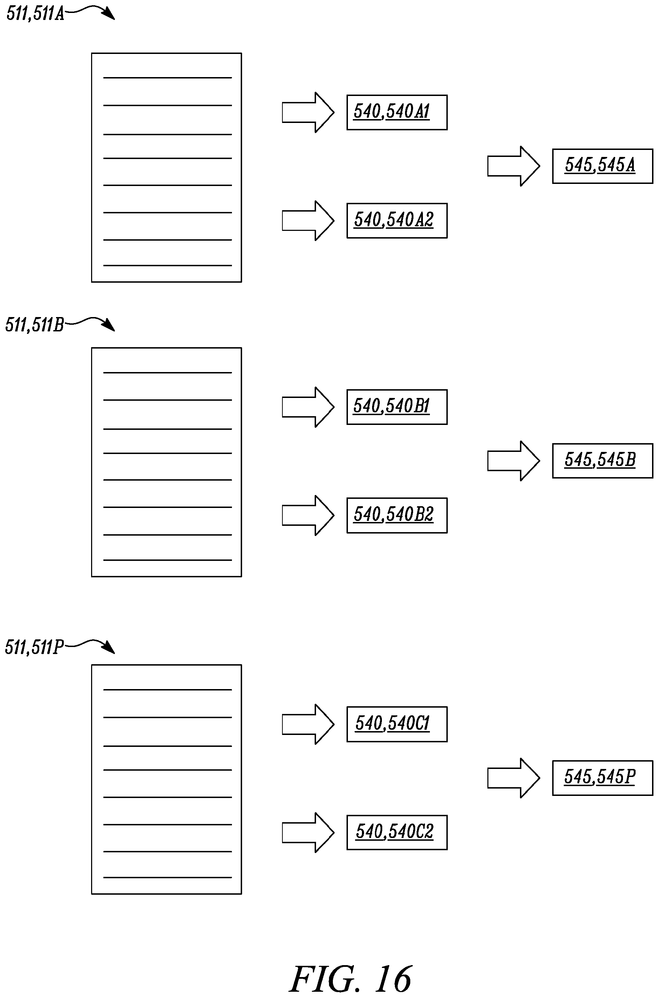

At step 609, the method 600 further includes aggregating, via the machine learning model, for each generated clinical note, the metric scores corresponding to the plurality of evaluation metrics to determine a model score associated with the note generation model that generated the generated clinical note. Referring to FIGS. 14 and 16, for example, the method 600 may include aggregating, via the machine learning model 562, for each generated clinical note 511, the metric scores 540 corresponding to the plurality of evaluation metrics to determine the model score 545 associated with the note generation model 561 that generated the generated clinical note 511.

At step 610, the method 600 further includes selecting one of the plurality of note generation models to generate clinical notes at least partially based on the model scores. Referring to FIGS. 14 and 16, for example, the method 600 may include selecting one of the plurality of note generation models 560 to generate clinical notes at least partially based on the model scores 545.

In some embodiments, the method 600 may include selecting the production note generation model having the highest model score to generate clinical notes instead of the production note generation model if the highest model score is greater than the model score of the production note generation model by a predetermined margin. Referring to FIGS. 14 and 16, for example, the method 600 may include selecting the note generation model 561 having the highest model score 545 to generate clinical notes instead of the production note generation model 561P if the highest model score 545 is greater than the model score 545P of the production note generation model 561P by the predetermined margin.

The method 600 may evaluate the plurality of note generation models based on the plurality of evaluation metrics with respect to the at least one final clinical note. In some examples, the at least one final clinical note may include a plurality of final clinical notes approved by a corresponding plurality of medical professionals. This may allow aggregating the model scores determined for the plurality of final clinical notes. Using the plurality of final clinical notes for evaluation may allow a more comprehensive evaluation of the plurality of note generation models, as different medical professionals may produce clinical notes in various different manners (writing style, formatting, terminology used, etc.).

In some embodiments, the at least one final clinical note is produced by amending one generated clinical note from the plurality of generated clinical notes. For example, the at least one final clinical note may be produced by amending the production clinical note.

The plurality of evaluation metrics may include at least one of a content factuality metric and a content coverage metric. For each generated clinical note, the metric score corresponding to each of the at least one of the content factuality metric and the content coverage metric may have a higher weight than the metric score of the rest of the plurality of evaluation metrics for determining the model score associated with the generated clinical note.

In examples where the at least one final clinical note is produced by amending the production clinical note, some metrics may exhibit a bias towards the production note generation model, that is, the model score for the production note generation model may be high. The content factuality metric and the content coverage metric may be less biased than others of the plurality of evaluation metrics for evaluation in such cases, and therefore may be preferably assigned higher aggregation weights for determining the model score for each note generation model. Thus, in such cases, it may be beneficial to rely more heavily on evaluation metrics such as the content factuality metric and the content coverage metric to obtain accurate model scores.

Further, some medical professionals may prefer using the note generation models that are determined to be more precise/conservative (i.e., having high model scores) to generate clinical notes, but others may prefer using the note generation models that create as much content as possible. To that end, in some examples, weighting of the plurality of evaluation metrics may be user-selected. As a result, the method 600 may select the note generation model that is best suited for a medical professional based on their preference. In some examples, the at least one final clinical note may be manually drafted by the medical professional from scratch. In such cases, the at least one final clinical note may also be used to adjust the weightage of the plurality of evaluation metrics, i.e., to fine-tune/calibrate the machine learning model.

In some embodiments, corresponding to the content factuality metric, the plurality of generated units of each set of generated units may include a plurality of generated SCUs and the plurality of final units of the at least one final clinical note may include a plurality of final SCUs. For the content factuality metric, the metric score for a respective generated clinical note from the plurality of generated clinical notes may be calculated by aggregating a plurality of generated entailment probabilities corresponding to the plurality of generated SCUs of the set of generated units associated with the respective generated clinical note. Each generated entailment probability from the plurality of generated entailment probabilities may be associated with a respective generated SCU and corresponds to a maximum entailment probability of the respective generated SCU with respect to the plurality of final SCUs of the at least one final clinical note.

In some embodiments, corresponding to the content coverage metric, the plurality of generated units of each set of generated units may include a plurality of generated SCUs and the plurality of final units of the at least one final clinical note may include a plurality of final SCUs. For the content coverage metric, the metric score for a respective generated clinical note from the plurality of generated clinical notes is calculated by aggregating a plurality of final entailment probabilities corresponding to the plurality of final SCUs of the at least one final clinical note. Each final entailment probability from the plurality of final entailment probabilities may be associated with a respective final SCU and corresponds to a maximum entailment probability of the respective final SCU with respect to the plurality of generated SCUs of set of generated SCUs corresponding to the generated clinical note.

The content factuality metric and the content coverage metric may be more reliable than other evaluation metrics in some cases, and thus may be assigned higher weights when calculating the model scores for the plurality of note generation models.

In some embodiments, the method 600 may further include receiving a plurality of second generated clinical notes. The plurality of second generated clinical notes may be generated by the corresponding plurality of note generation models based on a second DoPaCo transcript. The plurality of second generated clinical notes may include a second production clinical note that is generated by the production note generation model. Referring to FIGS. 14 and 17A, for example, the method 600 may include receiving the plurality of second generated clinical notes 710.

The method 600 may further include receiving at least one second final clinical note that is approved by the medical professional based on the second DoPaCo transcript. Referring to FIGS. 14 and 17A, for example, the method 600 may include receiving the at least one second final clinical note 720 that is approved by the medical professional based on the second DoPaCo transcript 750.

The method 600 may further include providing the plurality of second generated clinical notes and the at least one second final clinical note to the machine learning model. Referring to FIG. 14, for example, the method 600 may include providing the plurality of second generated clinical notes 710 and the at least one second final clinical note 720 to the machine learning model 562.

The method 600 may further include extracting, via the machine learning model, for each evaluation metric, a plurality of sets of second generated units from the plurality of second generated clinical notes. Each set of second generated units from the plurality of sets of second generated units is associated with a respective second generated clinical note from the plurality of second generated clinical notes. Each set of second generated units includes a plurality of second generated units. Referring to FIGS. 14 and 17A, for example, the method 600 may include extracting, via the machine learning model 562, for each evaluation metric, a plurality of sets of second generated units 730 from the plurality of second generated clinical notes 710.

The method 600 may further include extracting, via the machine learning model, for each evaluation metric, a plurality of second final units from the at least one second final clinical note. Referring to FIGS. 14 and 17B, for example, the method 600 may include extracting, via the machine learning model 562, for each evaluation metric, a plurality of second final units 725 from the at least one second final clinical note 720.

The method 600 may further include comparing, via the machine learning model, for each evaluation metric, the plurality of second generated units of each set of second generated units and the plurality of second final units of the at least one second final clinical note corresponding to the evaluation metric. Referring to FIGS. 14, 17A, and 17B, for example, the method 600 may include comparing, via the machine learning model 562, for each evaluation metric, the plurality of second generated units 715 of each set of second generated units 731 and the plurality of second final units 725 of the at least one second final clinical note 720 corresponding to the evaluation metric.

The method 600 may further include calculating, for each evaluation metric, a second metric score for each of the plurality of second generated clinical notes based on the comparison of the plurality of second generated units of each set of second generated units and the plurality of second final units of the at least one second final clinical note corresponding to the evaluation metric. Referring to FIGS. 14, 17A, and 18, the method 600 may include calculating, for each evaluation metric, the second metric score 740 for each of the plurality of second generated clinical notes 710.

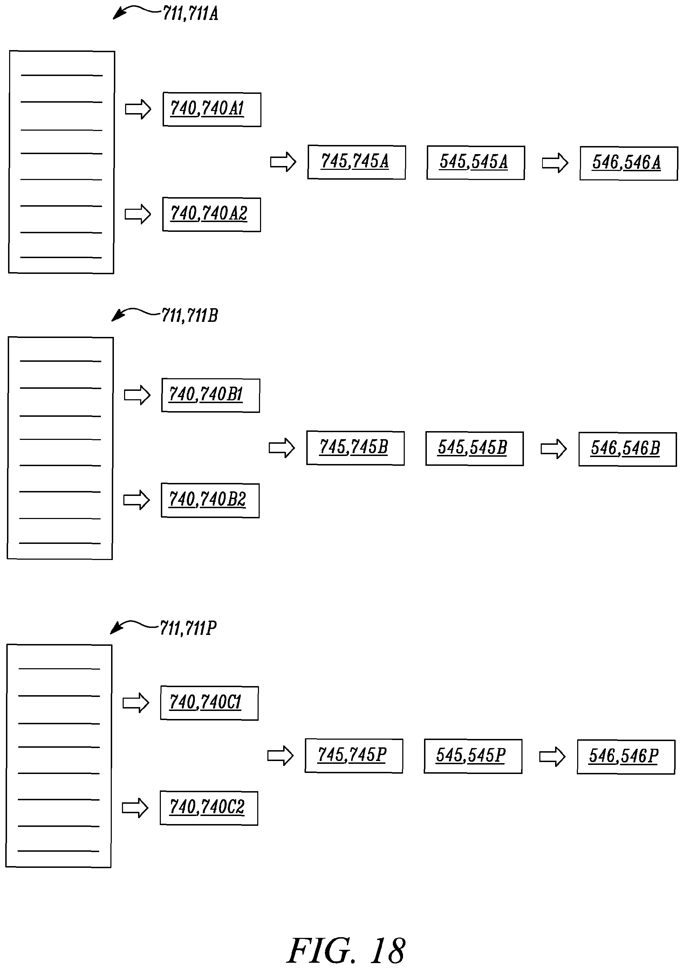

The method 600 may further include aggregating, via the machine learning model, for each second generated clinical note, the second metric scores corresponding to the plurality of evaluation metrics to determine a second model score associated with the note generation model that generated the second generated clinical note. Referring to FIGS. 14 and 18, for example, the method 600 may include aggregating, via the machine learning model 562, for each second generated clinical note 711, the second metric scores 740 corresponding to the plurality of evaluation metrics to determine the second model score 745 associated with the note generation model 561 that generated the second generated clinical note 711.

The method 600 may further include aggregating, for each note generation model, the model score and the second model score to determine an aggregated model score for the note generation model. Referring to FIGS. 14 and 18, for example, the method 600 may include aggregating, for each note generation model 561, the model score 545 and the second model score 745 to determine an aggregated model score 546 for the note generation model 561.

In some embodiments, selecting one of the plurality of note generation models to generate clinical notes is at least partially based on the aggregated model scores. Referring to FIGS. 14 and 18, for example, one of the plurality of note generation models 560 may be selected to generate clinical notes at least partially based on the aggregated model scores 546.

In some embodiments, the method 600 may include selecting the note generation model having the highest aggregated model score to generate clinical notes instead of the production note generation model if the highest aggregated model score is greater than the aggregated model score of the production note generation model by a predetermined margin. Referring to FIGS. 14 and 18, for example, the method 600 may include selecting the note generation model 561 having the highest aggregated model score 546 to generate clinical notes instead of the production note generation model 561P if the highest aggregated model score 546 is greater than the aggregated model score 546P of the production note generation model 561P by the predetermined margin.

The systems and techniques disclosed herein provide numerous advantages, including improving the predictive accuracy of the machine learning models trained using other techniques. For instance, over a set of 90 generated HPI sections corresponding to 90 Orthopedics DoPaCos, the estimations for the content factuality scores that are obtained by using the disclosed approach are on average 69% correlated with the corresponding content factuality scores. In contrast, an alternative ranking-based solution, trained using the approach disclosed in Liu, Yixin, Pengfei Liu, Dragomir Radev, and Graham Neubig. "BRIO: Bringing Order to Abstractive Summarization." In Proceedings of the 60th Annual Meeting of the Association for Computational Linguistics (Volume 1: Long Papers), pp. 2890-2903. 2022nl (hereinafter "BRIO") is on average 46% correlated with content factuality scores. In other words, the techniques of the disclosure result in an approximately 50% improvement to the Spearman correlation with content factuality scores. Results for content coverage scores follow the same trend, where the introduced approach obtains a correlation of 69 percent, while the ranking-based solution of certain conventional techniques, such as those disclosed in BRIO is on average only 39 percent correlated with content coverage scores, which is approximately a 76% improvement over conventional techniques. Similar improvements were observed for PE and A&P sections, where Spearman correlations with content factuality scores went from 52% to 75% for the PE section (44% improvement) and 37% to 72% for the A&P section (94% improvement) and Spearman correlations with content coverage scores increased from 54% to 81% for the PE section (50% improvement) and 55% to 73% for the A&P section (32% improvement).

As another example, our method for training a clinical-NLI model led to an improvement from 85.4 to 91.7 Macro-F1 score when compared to published techniques (e.g., Zhang and Bansal, "Finding a Balanced Degree of Automation for Summary Evaluation", 2021), as measured by accuracy against our ground-truth human annotations. Our techniques for decomposing text into fine-grained units in order to perform document-level NLI scoring lead to an improvement from 74.4% to 76.4% in terms of accuracy over the previously published techniques (e.g., Laban et al, "SummaC: Re-Visiting NLI-based Models for Inconsistency Detection in Summarization", 2022), which is approximately an 8% reduction in error rate.

It should also be appreciated that the techniques disclosed herein are inextricably linked to computing systems specifically adapted to generate and evaluate clinical notes and machine learning models capable of doing the same. For instance, the described systems and techniques generally must act in a time-constrained manner to achieve the desired results. For instance, in the context of a doctor-patient conversation, clinical notes generated from those conversations are only functionally useful in the short window between when the conversation has happened and the time the conversation must be documented. For example, if a doctor has a 20-minute conversation with the patient, the clinical note documenting exam results and other clinical findings need to be available essentially immediately to allow the captured information to be entered into the medical record or perform outcome planning. As a result, systems and techniques disclosed herein must be capable of operating in real-time or near real-time.

If, for example, this work was completed by hand determination of the quality of the clinical note via the same techniques disclosed herein, it could take potentially months to return the clinical notes that exceed a quality threshold back to the physician, therefore rendering the contents of clinical note largely useless as they would no longer be actionable due to a change in the patient's status, medical condition, and the like. While documentation delays for activities such as billing may be acceptable, delays in clinical note generation related to the care of the patient can impact medical care and removing those delays can help achieved improved medical outcomes. This means that the clinical note should be generated before the doctor completes charting on a given patient which often occurs during the course of the immediate or subsequent workday after the doctor patient/encounter. In contrast, given the large volume of data needed to utilize the techniques disclosed herein, it would take a human many weeks or months to perform the steps necessary to analyze the clinical notes as contemplated by this disclosure, and these delays would fundamentally undermine the purpose of these computerized systems.

The systems and the computer-implemented methods of the present disclosure may evaluate the quality of AI generated clinical notes and perform one or more actions on the AI generated clinical notes based on the evaluated quality. The systems and the computer-implemented methods of the present disclosure may also evaluate a quality of note generation models that generate clinical notes, and select the machine learning model best suited for specific user based on their preferences.

Thus, aspects of the disclosure can select a model on a person-by-person or physician-by-physician basis to improve the note generation capabilities of the system relative to each physician. That is, over time, the model can learn the preferences of each physician to generate more accurate clinical notes. In general, it should be understood that these preferences are not necessarily explicitly expressed, but implicitly indicated through the provided final notes. The effect is that it requires a large number (e.g., hundreds or thousands) of ever-changing final note examples to compute the discussed metrics. And these metrics must be computed again and again for automatically generated notes for existing production and candidate models. Furthermore, these computations need to be done for each physician where a personal model might be selected. None of the above is feasible to be done by a human (with or without the aid of pen and paper) within a reasonable amount of effort. For instance, commercial products need to scale to several hundred thousand physicians, making it impracticable for any number of humans to manually perform the techniques of this disclosure to obtain the improvements and advantages disclosed herein. In short, the systems and techniques of this disclosure are inextricably linked to the realm of computerized clinical note generation.

Unless otherwise indicated, all numbers expressing feature sizes, amounts, and physical properties used in the specification and claims are to be understood as being modified by the term "about." Accordingly, unless indicated to the contrary, the numerical parameters set forth in the foregoing specification and attached claims are approximations that can vary depending upon the desired properties sought to be obtained by those skilled in the art utilizing the teachings disclosed herein.

Although specific embodiments have been illustrated and described herein, it will be appreciated by those of ordinary skill in the art that a variety of alternate and/or equivalent implementations can be substituted for the specific embodiments shown and described without departing from the scope of the present disclosure. This application is intended to cover any adaptations or variations of the specific embodiments discussed herein. Therefore, it is intended that this disclosure be limited only by the claims and the equivalents thereof.

## Claims

1. A system for evaluating generated clinical notes, the system comprising:
one or more computer processors; and
at least one non-transitory computer-readable storage, communicatively coupled to the one or more computer processors, having stored thereon a note generation model, a machine learning model, and instructions that when executed by the one or more computer processors cause the one or more computer processors to:
receive a generated clinical note associated with a patient and a doctor-patient conversation (DoPaCo) transcript based on which the generated clinical note is generated;
provide the DoPaCo transcript to the note generation model;
generate, via the note generation model, a plurality of pseudo-reference clinical notes based on the DoPaCo transcript;
select at least one evaluation metric for evaluating the generated clinical note;
extract, via the machine learning model, for each evaluation metric from the at least one evaluation metric, a plurality of generated units from the generated clinical note;
extract, via the machine learning model, for each evaluation metric, a plurality of sets of pseudo units from the plurality of pseudo-reference clinical notes, wherein each set of pseudo units from the plurality of sets of pseudo units is associated with a respective pseudo-reference clinical note from the plurality of pseudo-reference clinical notes, and wherein each set of pseudo units comprises a plurality of pseudo units;
compare, via the machine learning model, for each evaluation metric, the plurality of generated units and the plurality of pseudo units of each set of pseudo units corresponding to the evaluation metric;
calculate, for each evaluation metric, a plurality of sub-scores for the generated clinical note based on the comparison of the plurality of generated units and the plurality of pseudo units of each set of pseudo units corresponding to the evaluation metric, wherein each sub-score from the plurality of sub-scores corresponding to a respective evaluation metric is associated with a respective pseudo-reference clinical note;
aggregate, for each evaluation metric, the plurality of sub-scores corresponding to the evaluation metric to calculate a metric score corresponding to the evaluation metric;
aggregate, via the machine learning model, the metric score corresponding to each of the at least one evaluation metric to predict a quality score of the generated clinical note; and
output, via an output device:
the generated clinical note if the quality score is greater than or equal to a predetermined acceptable threshold; and
a request to manually draft a drafted clinical note based on the DoPaCo transcript if the quality score is less than the predetermined acceptable threshold.

2. The system of claim 1, wherein the at least one evaluation metric comprises a clinical concept precision metric, wherein, corresponding to the clinical concept precision metric, the plurality of generated units comprises a plurality of generated clinical concepts and the plurality of pseudo units of each set of pseudo units comprises a plurality of pseudo clinical concepts, and wherein, for the clinical concept precision metric, each sub-score associated with the respective pseudo-reference clinical note is calculated based on a ratio of (i) a number of the plurality of generated clinical concepts that match with the plurality of pseudo clinical concepts of the set of pseudo units corresponding to the respective pseudo-reference clinical note to (ii) a total number of the plurality of generated clinical concepts of the generated clinical note.

3. The system of any of claims 1 to 2, wherein the at least one evaluation metric comprises a clinical concept coverage metric, wherein, corresponding to the clinical concept coverage metric, the plurality of generated units comprises a plurality of generated clinical concepts and the plurality of pseudo units of each set of pseudo units comprises a plurality of pseudo clinical concepts, and wherein, for the clinical concept coverage metric, each sub-score associated with the respective pseudo-reference clinical note is calculated based on a ratio of (i) a number of the plurality of pseudo clinical concepts of the set of pseudo units corresponding to the respective pseudo-reference clinical note that match with the plurality of generated clinical concepts of the generated clinical note to (ii) a total number of the plurality of pseudo clinical concepts of the set of pseudo units corresponding to the respective pseudo-reference clinical note.

4. The system of any of claims 1 to 3, wherein the at least one evaluation metric comprises a content factuality metric, wherein, corresponding to the content factuality metric, the plurality of generated units comprises a plurality of generated semantic content units (SCUs) and the plurality of pseudo units of each set of pseudo units comprises a plurality of pseudo SCUs, and wherein, for the content factuality metric, each sub-score associated with the respective pseudo-reference clinical note is calculated by aggregating a plurality of generated entailment probabilities corresponding to the plurality of generated SCUs, wherein each generated entailment probability from the plurality of generated entailment probabilities is associated with a respective generated SCU and corresponds to a maximum entailment probability of the respective generated SCU with respect to the plurality of pseudo SCUs of the respective pseudo-reference clinical note.

5. The system of any of claims 1 to 4, wherein the at least one evaluation metric comprises a content coverage metric, wherein, corresponding to the content coverage metric, the plurality of generated units comprises a plurality of generated semantic content units (SCUs) and the plurality of pseudo units of each set of pseudo units comprises a plurality of pseudo SCUs, and wherein, for the content coverage metric, each sub-score associated with the respective pseudo-reference clinical note is calculated by aggregating a plurality of pseudo entailment probabilities corresponding to the plurality of pseudo SCUs of the respective pseudo-reference clinical note, wherein each pseudo entailment probability from the plurality of pseudo entailment probabilities is associated with a respective pseudo SCU and corresponds to a maximum entailment probability of the respective pseudo SCU with respect to the plurality of generated SCUs of the generated clinical note.

6. The system of any of claims 1 to 5, wherein the instructions further cause the one or more computer processors to:
calculate, for one evaluation metric from the at least one evaluation metric, a plurality of unit scores for the plurality of generated units of the generated clinical note based on the comparison of the plurality of generated units and the plurality of pseudo units of each set of pseudo units corresponding to the one evaluation metric, wherein each unit score from the plurality of unit scores corresponds to a respective generated unit from the plurality of generated units;
determine which of the plurality of generated units have the unit scores less than a predetermined unit threshold;
determine one or more portions of the generated clinical note corresponding to the plurality of generated units having the unit scores less than the predetermined unit threshold; and
highlight the one or more portions of the generated clinical note corresponding to the plurality of generated units having the unit scores less than the predetermined unit threshold prior to outputting the generated clinical note.

7. The system of any of claims 1 to 6, wherein the instructions further cause the one or more computer processors to:
receive a plurality of transcript confidence scores corresponding to a plurality of transcript units of the DoPaCo transcript, wherein each transcript confidence score from the plurality of transcript confidence scores corresponds to a respective transcript unit from the plurality of transcript units;
determine, via the machine learning model, for one or more evaluation metrics from the at least one evaluation metric, which of the plurality of generated units correspond to which of the plurality of transcript units; and
assign, via the machine learning model, for the one or more evaluation metrics, a plurality of transcript weights to the plurality of generated units based on the plurality of transcript confidence scores,
wherein, for one or more evaluation metrics, the plurality of sub-scores is calculated further based on the plurality of transcript weights.

8. The system of any of claims 1 to 7, wherein the at least one non-transitory computer-readable storage further stores a knowledge graph of clinical data associated with the patient, and wherein the instructions further cause the one or more computer processors to assign, for one or more evaluation metrics from the at least one evaluation metric, a plurality of knowledge weights to the plurality of generated units based on the knowledge graph, and wherein, for the one or more evaluation metrics, the plurality of sub-scores is calculated further based on the plurality of knowledge weights.

9. The system of any of claims 1 to 8, wherein the generated clinical note comprises a plurality of generated note sections and each of the plurality of pseudo-reference clinical notes comprises a plurality of pseudo note sections corresponding to the plurality of generated note sections, and wherein the instructions further cause the one or more computer processors to:
compare, via the machine learning model, for each evaluation metric, the plurality of generated units of each generated note section and the plurality of pseudo units of a respective pseudo note section corresponding to the generated note section of each set of pseudo units;
calculate, for each evaluation metric, a plurality of section sub-scores for each generated note section of the generated clinical note based on the comparison of the plurality of generated units of each generated note section and the plurality of pseudo units of the respective pseudo note section of each set of pseudo units, wherein, each section sub-score from the plurality of section sub-scores corresponding to a respective evaluation metric is associated with a respective pseudo-reference clinical note; and
aggregate, for each evaluation metric, the plurality of section sub-scores for each generated note section to calculate a plurality of section scores for the plurality of generated note sections, wherein each generated note section has a corresponding section score from the plurality of section scores;
wherein, for each evaluation metric, the plurality of section scores is aggregated with the plurality of sub-scores to calculate the metric score.

10. The system of any of claims 1 to 9, wherein the instructions further cause the one or more computer processors to modify at least one generated note section from the plurality of generated note sections of the generated clinical note based on the section score of the at least one generated note section prior to outputting the generated clinical note, wherein modifying the at least one generated note section comprises:
removing the at least one generated note section from the generated clinical note if the section score is less than a predetermined acceptable section threshold; and
highlighting the at least one generated note section of the generated clinical note if the section score is greater than or equal to the predetermined acceptable section threshold.

11. The system of any of claims 1 to 10, wherein the instructions further cause the one or more computer processors to:
compare, via the machine learning model, for each evaluation metric, the plurality of pseudo units of the plurality of sets of pseudo units with each other;
determine, for each evaluation metric, a plurality of missing pseudo units from the plurality of pseudo units based on the comparison of the plurality of pseudo units of the plurality of sets of pseudo units with each other, wherein the plurality of missing pseudo units comprises the pseudo units that are missing equivalent generated units from the plurality of generated units and that repeat among the plurality of sets of pseudo units; and
add the plurality of missing pseudo units to the generated clinical note before outputting the generated clinical note if the quality score is greater than or equal to the predetermined acceptable threshold.

12. The system of any of claims 1 to 11, wherein the instructions further cause the one or more computer processors to:
receive the drafted clinical note;
extract, via the machine learning model, for each evaluation metric, a plurality of drafted units from the drafted clinical note;
compare, via the machine learning model, for each evaluation metric, the plurality of generated units and the plurality of drafted units corresponding to the evaluation metric;
calculate, for each evaluation metric, a draft reference metric score based on the comparison of the plurality of generated units and the plurality of drafted units corresponding to the evaluation metric;
aggregate, via the machine learning model, the draft reference metric score corresponding to each of the at least one evaluation metric to determine a draft reference quality score of the generated clinical note;
update the machine learning model based on the quality score and the draft reference quality score; and
output the updated machine learning model to at least one of a storage device and the non-transitory computer-readable storage.

13. The system of any of claims 1 to 12, wherein the instructions further cause the one or more computer processors to
receive an amended clinical note, wherein the amended clinical note is produced by amending the generated clinical note;
extract, via the machine learning model, for each evaluation metric, a plurality of amended units from the amended clinical note;
compare, via the machine learning model, for each evaluation metric, the plurality of generated units and the plurality of amended units corresponding to the evaluation metric;
calculate, for each evaluation metric, an amended reference metric score based on the comparison of the plurality of generated units and the plurality of amended units corresponding to the evaluation metric;
aggregate, via the machine learning model, the amended reference metric score corresponding to each of the at least one evaluation metric to determine an amended reference quality score of the generated clinical note;
update the machine learning model based on the quality score and the amended reference quality score; and
output the updated machine learning model to at least one of a storage device and the non-transitory computer-readable storage.

14. The system of any of claims 1 to 13, wherein the at least one evaluation metric comprises at least one of a content factuality metric and a content coverage metric, and wherein the amended reference metric score corresponding to each of the at least one of the content factuality metric and the content coverage metric has a higher weight than the amended reference metric score of the rest of the at least one evaluation metric for determining the amended reference quality score of the generated clinical note.
